# EUROPEAN PATENT APPLICATION

(11) **EP 4 684 751 A1**
(43) Date of publication of application: **28.01.2026**
(21) Application number: 24774259.6
(22) Date of filing: 22.03.2024
(51) Int. Cl.: A61B 34/35, A61B 34/37, A61B 34/30, B25J 3/00

(54) **SURGICAL ROBOT, CONTROL METHOD THEREFOR, SYSTEM, AND MEDIUM**

(30) Priority: 23.03.2023 CN 202310326250; 23.03.2023 CN 202310332581; 23.03.2023 CN 202310326480
(71) Applicant: Shenzhen Edge Medical Co., Ltd, Shenzhen, Guangdong 518000 (CN)
(72) Inventor: YAN, YuCheng, Shenzhen, Guangdong 518000 (CN); LU, RenCai, Shenzhen, Guangdong 518000 (CN); CHEN, Feng, Shenzhen, Guangdong 518000 (CN); LIN, MinCai, Shenzhen, Guangdong 518000 (CN); YE, GuoQiang, Shenzhen, Guangdong 518000 (CN); GAO, YuanQian, Shenzhen, Guangdong 518000 (CN); LI, Shuai, Shenzhen, Guangdong 518000 (CN); SUN, Qiang, Shenzhen, Guangdong 518000 (CN); MAO, JianLe, Shenzhen, Guangdong 518000 (CN)
(74) Representative: Metida
(86) International application number: PCT/CN2024/083320
(87) International publication number: WO 2024/193696

(57) **Abstract**

The present disclosure discloses a surgical robot and a controlling method, a system, and a medium. The surgical robot includes: a mechanical arm and a control device, where an end of the mechanical arm is provided with an instrument holder for installing multiple surgical instruments. The control device is configured to control movement of the mechanical arm so that the multiple surgical instruments rotate around the same remote center of motion, and: in response to a boundary setting instruction, set the current posture of the instrument holder as the motion boundary for the instrument holder's movement around the remote center of motion; in response to the instrument holder rotating around the remote center of motion to the motion boundary, control the instrument holder to move in a preset motion mode. Using the solution of the present disclosure, the function of intraoperatively preventing the mechanical arm from squeezing the patient is added to the surgical robot, avoiding secondary injury to the patient caused by abnormal collisions, and improving safety and reliability of the surgical robot's operation.

## Description

### Technical Field

The present disclosure relates to medical instruments, more particularly to a surgical robot and a control method, a system and a medium.

### BACKGROUND

With development of technology and innovation of artificial intelligence, a surgical robot is beginning to be widely used in clinical surgery. The surgical robot may perform actual surgery instead of a doctor. The doctor needs to operate the surgical robot from a remote master console, thereby serving patients in more areas, such as remote areas lacking experienced surgeons.

In practical applications, the mechanical arm is positioned by medical staff and docked with a cannula (cannula), then surgical instruments are installed. The surgical instruments are inserted into the patient's body through the cannula, and then a lead surgeon performs the surgery.

On one hand, after the mechanical arm is docked with the cannula and before the surgical instruments are installed, a mis-operation by the medical staff may cause an instrument holding component at the end of the mechanical arm to squeeze skin of the patient, causing a secondary injury to the patient.

On the other hand, after the instruments are installed and the lead surgeon begins the surgery, since the lead surgeon performs the actual surgical operation by watching the image view on the remote master console, which presents the surgical field inside the patient, the lead surgeon cannot see actual movement of the mechanical arm outside the patient. However, the movable workspace of the mechanical arm of the surgical robot is much larger than the workspace required for actual surgical operations. Therefore, without any external feedback, there is a risk of the mechanical arm contacting or even squeezing the patient while the lead surgeon operates the surgical robot, which will cause the secondary injury to the patient.

### SUMMARY

To solve the problem in the prior art that the mechanical arm of a surgical robot risks contacting or even squeezing the patient, the present disclosure provides the following technical solution:
In one aspect, the present disclosure provides a surgical robot, comprising: a mechanical arm and a control device, wherein an end of the mechanical arm is provided with an instrument holder for installing a plurality of surgical instruments, the control device is configured to control the mechanical arm to move and enable the plurality of surgical instruments rotate around the same remote center of motion, and:
in response to a boundary setting instruction, set a current posture of the instrument holder as a motion boundary when the instrument holder moves around the remote center of motion;
in response that the instrument holder rotates around the remote center of motion to the motion boundary, control the instrument holder to move in a preset motion mode.

In another aspect, the present disclosure provides an anti-squeezing control method for the mechanical arm of the surgical robot according to any one of the above, comprising:
in response to a boundary setting instruction, setting a current posture of the instrument holder as a motion boundary when the instrument holder moves around the remote center of motion;
in response to the instrument holder rotating around the remote center of motion to the motion boundary, controlling the instrument holder to move in a preset motion mode.

In yet another aspect, the present disclosure provides a computer-assisted surgical system, comprising the surgical robot according to any one of the above and a console for remotely controlling the surgical robot.

In still another aspect, the present disclosure provides a readable storage medium, the readable storage medium stores a computer program, which when executed by a processor, implements the steps of the anti-squeezing control method for the mechanical arm of the surgical robot described above.

The beneficial effects of the present disclosure are: using the solution of the present disclosure, the function of intraoperatively preventing the mechanical arm from squeezing the patient is added to the surgical robot, avoiding secondary injury to the patient caused by abnormal collisions, and improving the safety and reliability of the surgical robot's operation.

### Brief Description of Drawings

FIG. 1 is a schematic diagram of a usage scenario of an embodiment of a surgical robot of the present disclosure.
FIG. 2 is a schematic diagram of an overall structure of an embodiment of the surgical robot of the present disclosure.
FIG. 3 is a schematic diagram of a kinematic model of an embodiment of the mechanical arm of the surgical robot of the present disclosure.
FIG. 4 is a schematic diagram of arrangement of an embodiment of a force sensor of the present disclosure.
FIG. 5 is a schematic diagram of the structure of an embodiment of an instrument holder of the present disclosure.
FIG. 6A is a schematic diagram of a scenario of an embodiment of a cannula inserted into a human body of the present disclosure.
FIG. 6B is a schematic diagram of a scenario of an embodiment of a cannula inserted into a human body of the present disclosure.
FIG. 7A is a schematic diagram of a scenario of an embodiment of a surgical instrument inserted into a human body of the present disclosure.
FIG. 7B is a schematic diagram of a scenario of an embodiment of a surgical instrument inserted into a human body of the present disclosure.
FIG. 7C is a schematic diagram of a scenario of an embodiment of a surgical instrument inserted into a human body of the present disclosure.
FIG. 8A is a schematic diagram of a scenario of an embodiment of a surgical instrument inserted into a human body of the present disclosure.
FIG. 8B is a schematic diagram of a scenario of an embodiment of a surgical instrument inserted into a human body of the present disclosure.
FIG. 8C is a schematic diagram of a scenario of an embodiment of a surgical instrument inserted into a human body of the present disclosure.
FIG. 9A is a main flowchart of an embodiment of a cannula docking control method for the surgical robot of the present disclosure.
FIG. 9B is a sub-flowchart of an embodiment of the cannula docking control method for the surgical robot.
FIG. 10A is a flowchart of an embodiment of the cannula docking control method for the surgical robot.
FIG. 10B is a flowchart of an embodiment of the cannula docking control method for the surgical robot.
FIG. 11 is a schematic diagram of the arrangement of an embodiment of a force sensor of the present disclosure.
FIG. 12 is a flowchart of an embodiment of a method for setting motion limits for the surgical robot of the present disclosure.
FIG. 13 is a flowchart of an embodiment of an anti-squeezing control method for the mechanical arm of the surgical robot of the present disclosure.
FIG. 14 is a flowchart of an embodiment of motion control for the mechanical arm of the surgical robot of the present disclosure.
FIG. 15 is a flowchart of an embodiment of a manual setting control method for the person-squeezing prevention function of the mechanical arm of the surgical robot of the present disclosure.
FIG. 16 is a flowchart of an embodiment of an automatic detection control method for the person-squeezing prevention function of the mechanical arm of the surgical robot of the present disclosure.
FIG. 17A is a front view of the retracted position of an embodiment of the mechanical arm of the surgical robot of the present disclosure.
FIG. 17B is a top view of the retracted position of an embodiment of the mechanical arm of the surgical robot of the present disclosure.
FIG. 18A is a front view of a drape deployment position of an embodiment of the mechanical arm of the surgical robot of the present disclosure.
FIG. 18B is a top view of a drape deployment position of an embodiment of the mechanical arm of the surgical robot of the present disclosure.
FIG. 19A is a front view of a right docking position of an embodiment of the mechanical arm of the surgical robot of the present disclosure.
FIG. 19B is a top view of the right docking position of an embodiment of the mechanical arm of the surgical robot of the present disclosure.
FIG. 20A is a front view of a left docking position of an embodiment of the mechanical arm of the surgical robot of the present disclosure.
FIG. 20B is a top view of a left docking position of an embodiment of the mechanical arm of the surgical robot of the present disclosure.
FIG. 21 is a flowchart of an embodiment of a positioning control method for the mechanical arm of the surgical robot of the present disclosure.
FIG. 22 is a flowchart of an embodiment of motion control for the mechanical arm of the surgical robot of the present disclosure.
FIG. 23 is a flowchart of an embodiment of a collision detection method for the mechanical arm of the surgical robot of the present disclosure.
FIG. 24 is a schematic diagram of the structure of the hardware operating environment involved in the various methods of the surgical robot of the present disclosure.

### Detailed Description

To facilitate the understanding of the present disclosure, the following describes the present disclosure in more detail with reference to accompanying drawings and specific embodiments. The accompanying drawings show preferred embodiments of the present disclosure. However, the present disclosure may be implemented in many different forms and is not limited to the embodiments described herein. On the contrary, a purpose of providing these embodiments is to make the understanding of the disclosure of the present disclosure more thorough and comprehensive.

It should be noted that unless otherwise defined, all technical and scientific terms used herein have the same meaning as commonly understood by one of ordinary skill in the art to which the present disclosure belongs. The terms used in the description of the present disclosure are for the purpose of describing specific embodiments only and are not intended to limit the present disclosure. For example, the term "multiple" includes two or more.

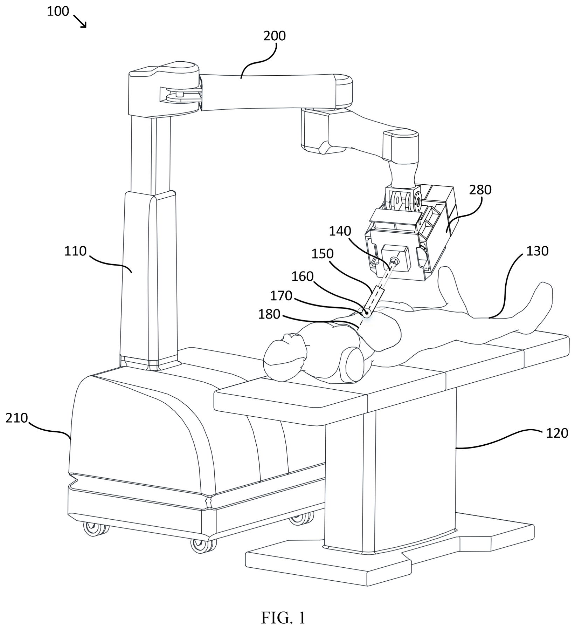

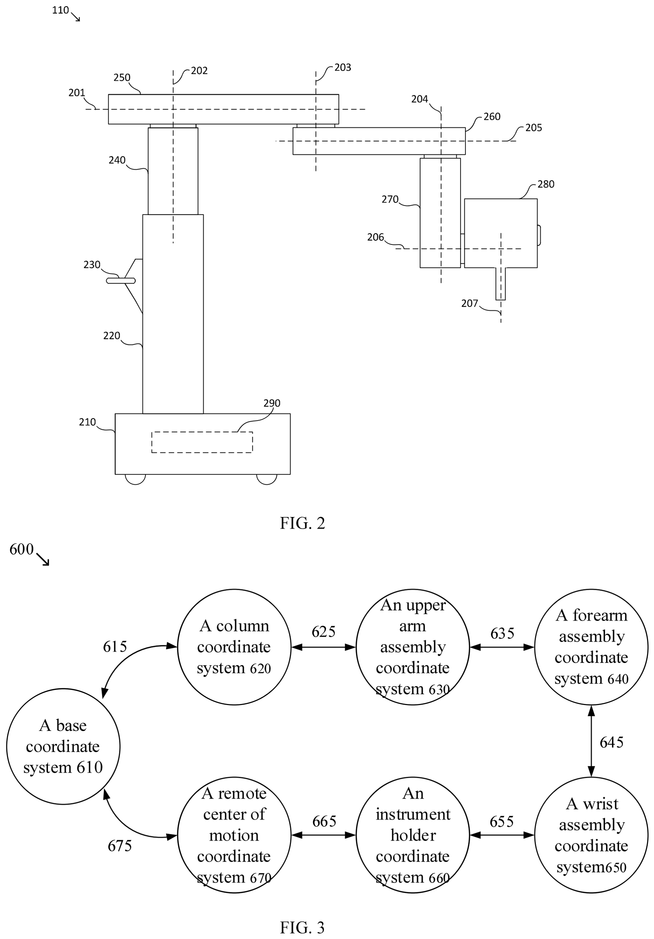

Referring to Fig. 1, Fig. 1 is a schematic diagram of a usage scenario 100 of an embodiment of the surgical robot 110 of the present disclosure. As shown in Fig. 1, in one aspect, the present disclosure provides a surgical robot 110, includes: a chassis 210, a mechanical arm 200, an instrument holder 280, and a control device 290 (not shown in Fig. 1, refer to Fig. 2), where a proximal end of the mechanical arm 200 is connected to the chassis 210, a distal end of the mechanical arm 200 is connected to the instrument holder 280, the instrument holder 280 is used to install the surgical instrument and dock with a cannula 150, the instrument holder 280 is configured to rotate around a remote center of motion 160, the control device 290 is used to control the mechanical arm 200 to perform docking movement so that the docking device 140 on the instrument holder 280 successfully docks with the cannula 150.

After docking, the medical staff installs the surgical instrument to the instrument holder 280 and inserts an end of the surgical instrument into the human body through the cannula 150. Then the lead surgeon controls the surgical instrument through the remote console to perform the surgery.

Specifically, the surgical robot 110 is pushed to a position near a surgical bed 120 during preoperative preparation process. After the doctor creates an incision 170 and bores a hole in the patient 130, a portion of the cannula 150 used to support the surgical instrument will be inserted into the patient's body 130 through the incision 170. After sealing is done, the medical staff will manually drag the surgical robot 110 to complete an assembly and docking with an end of the cannula 150 that has been inserted into the patient's body 130. This ensures that during the doctor's operation of the surgical robot 110 from the master console, the mechanical arm 200 may perform fixed-point rotation around a remote center of motion 160 on a central axis 180 of the cannula 150 without causing any additional damage to the patient's incision 170.

Among, them, the remote center of motion 160 is a virtual fixed point. After the cannula 150 and the instrument holder 280 complete preliminary docking, the remote center of motion 160 is located on the central axis 180 of the cannula 150 and is flush with the patient's incision 170. At this time, since the cannula 150 and the instrument holder 280 are fixedly connected, the instrument holder 280 may be controlled to perform fixed-point rotation around the remote center of motion, also driving the cannula 150 to perform fixed-point rotation around the remote center of motion. This facilitates multi-angle operation of the surgical instrument passing through the cannula 150 inside the human body.

The mechanical arm 200 of the surgical robot 110 includes multiple links and joints to adapt to various distance and angle adjustments during surgery.

Specifically, referring to Fig. 2, Fig. 2 is a schematic diagram of the overall structure of an embodiment of the surgical robot 110 of the present disclosure. As shown in Fig. 2, the surgical robot 110 further comprises a chassis 210, a push handle 230, a main support column 220, a lifting column 240, an upper arm assembly 250, a forearm assembly 260, a wrist assembly 270, an instrument holder 280, and a control device 290. The mechanical arm 200 of the surgical robot 110 includes the lifting column 240, upper arm assembly 250, forearm assembly 260, wrist assembly 270, and instrument holder 280. The control device 290 can be located inside the chassis 210.

Among them, the chassis 210 has a wheeled structure. An operator may control movement of the chassis 210 through the handle 230, enabling transportation of the surgical robot 110. The main support column 220 is fixedly connected to the chassis 210, providing support for the overall structure of the surgical robot 110. The lifting column 240 may move up and down relative to the main support column 220 along its central axis 202, enabling the mechanical arm 200 to be raised or lowered. The upper arm assembly 250 may perform rotational movement around the central axis 202 of the lifting column 240. The forearm assembly 260 and the upper arm assembly 250 have a rotational joint at a pivot axis 203, allowing rotational movement. Similarly, the wrist assembly 270 may perform rotational movement around the pivot axis 204 of the forearm assembly 260. The instrument holder 280 may perform rotational movement around the pivot axis 206 of the wrist assembly 270. The central axes 202, 203, and 204 are parallel to each other and perpendicular to the horizontal plane, and are spatially perpendicular to the pivot axis 206. The central axis 207 of the instrument holder 280 passes through the remote center of motion 160 and is perpendicular to the pivot axis 206. The horizontal central plane 201 of the upper arm assembly 250 and the horizontal central plane 205 of the forearm assembly 260 are parallel to each other and perpendicular to the central axis 207 of the instrument holder 280 and the central axis 201. After the cannula 150 is docked to the instrument holder 280, the central axis 207 of the instrument holder 280 substantially coincides with a central axis 180 of the cannula 150. The combined movement of the various moving parts 240-280 enables the initial positioning of the mechanical arm 200 and the movement of the remote center of motion 160 at the end of the mechanical arm 200 and the rotational movement of the cannula 150.

Fig. 3 is a schematic diagram of a kinematic model 600 of an embodiment of the mechanical arm 200 of the surgical robot 110 of the present disclosure. As shown in Fig. 3, the kinematic model 600 includes kinematic information associated with many active devices. The kinematic information is based on a known kinematic model for the various mechanism links and joints of the surgical robot 110. The kinematic information is further based on information related to the positions and postures of the joints of the surgical robot 110, measured using one or more position sensors (e.g., encoders) to obtain linear position of prismatic joints and the rotational position of revolute joints. The kinematic model 600 includes several coordinate systems or frames and coordinate transformation relationships (e.g., homogeneous transformations) to transform the description of an object's position and posture from one coordinate system to another. The kinematic model 600 may use one or several of involved coordinate systems and transformation relationships through forward or inverse transformations to establish kinematic relationships between adjacent or non-adjacent coordinate systems. In some embodiments, the kinematic model 600 is used to model the kinematic relationships of the surgical robot 110 in Fig. 1.

The kinematic model 600 includes a base coordinate system 610 for kinematically modeling positions and postures of the joints of the surgical robot 110. The base coordinate system 610 is established on the chassis and remains relatively stationary to the chassis. In some embodiments, the base coordinate system 610 serves as a reference point for modeling between the motion joints of the surgical robot 110. Once determined the base coordinate system 610, a geometric relationship of the origin and axis orientation on the chassis mechanical structure is fixed. Furthermore, to facilitate an intuitive description of motion information of each joint, a Z-axis orientation of the base coordinate system 610 may be perpendicular to ground or floor surface where the surgical robot 110 is placed.

The kinematic model 600 further includes a column coordinate system 620, used to model position and posture of the lifting column 240, and the column coordinate system 620 remains relatively stationary to the lifting column 240 that is allowed to move. In some embodiments, considering convenience and speed of establishing transformation relationships between adjacent coordinate systems, and based on the rules for establishing coordinate systems, the Z-axis of the column coordinate system 620 may coincide with the central axis 202 of the lifting column 240. The X and Y axes of the column coordinate system 620 may be parallel to axes of the base coordinate system 610. The origin of the column coordinate system 620 may be determined at an intersection of a central axis 202 of the lifting column and the horizontal central plane 201 of the upper arm assembly 250. In some embodiments, one or more sensors may be used to measure translational position of the lifting column 240 relative to the fixed main support column 220, and this is further used to determine the coordinate transformation relationship 615 between the column coordinate system 620 and the base coordinate system 610 of the chassis.

The kinematic model 600 further includes an upper arm assembly coordinate system 630 established at an end of the upper arm assembly 250 in the mechanical arm 200. In some embodiments, the upper arm assembly coordinate system 630 may be established at an intersection of the horizontal central plane 201 of the upper arm assembly 250 and a central axis 203 of the forearm assembly 260, used to describe the motion information of the upper arm assembly 250. Based on the rules for establishing coordinate systems, the Z-axis of the upper arm assembly coordinate system 630 can coincide with the central axis 203 of the forearm assembly 260. In some embodiments, one or more sensors may be used to measure the rotational position of the upper arm assembly 250 relative to the lifting column 240, and this is further used to determine the coordinate transformation relationship 625 between the upper arm assembly coordinate system 630 and the column coordinate system 620. In some embodiments, based on the coordinate transformation relationships 615 and 625, a kinematic model between the upper arm assembly coordinate system 630 and the base coordinate system 610 may be established, and the kinematic model may further be used to describe motion information of the upper arm assembly 250 in the base coordinate system 610. The motion information includes position information and posture information. In some embodiments, based on the motion information of the upper arm assembly 250 in the base coordinate system 610 and the transformation relationships 615 and 625, through inverse kinematics, the motion information of the joints of the lifting column 240 and upper arm assembly 250 may be determined.

The kinematic model 600 further includes a forearm assembly coordinate system 640 established at the end of the forearm assembly 260. In some embodiments, the forearm assembly coordinate system 640 may be established at an intersection of the horizontal central plane 205 of the forearm assembly 260 and the central axis 204 of the wrist assembly 270, used to describe the motion information of the forearm assembly 260. Based on the rules for establishing coordinate systems, the Z-axis of the forearm assembly coordinate system 640 may coincide with the central axis 204 of the wrist assembly 270. In some embodiments, one or more sensors may be used to measure the rotational position of the forearm assembly 260 relative to the upper arm assembly 250, and this is further used to determine the coordinate transformation relationship 635 between the forearm assembly coordinate system 640 and the upper arm assembly coordinate system 630. In some embodiments, based on the transformation relationships 625 and 635, a kinematic model between the forearm assembly coordinate system 640 and the column coordinate system 620 may be established, and this may further be used to describe posture motion information of the forearm assembly 260 relative to the lifting column 240. In some embodiments, based on the combination of transformation relationships 615, 625, and 635, a kinematic model between coordinate systems 640 and 610 may be established, and the kinematic model may further be used to describe the motion information of the forearm assembly 260 in the base coordinate system 610. The motion information includes position information and posture information. In some embodiments, based on the motion information of the forearm assembly 260 in the base coordinate system 610 and the transformation relationships 615, 625, and 635, through inverse kinematics, the motion information of the joints of the lifting column 240, upper arm assembly 250, and forearm assembly 260 may be determined.

The kinematic model 600 further includes a wrist assembly coordinate system 650 established at the end of the wrist assembly 270. In some embodiments, the wrist assembly coordinate system 650 may be established at an intersection of the central axis 204 of the wrist assembly 270 and a central axis 207 of the instrument holder, used to describe motion information of the wrist assembly 270. Based on the rules for establishing coordinate systems, the Z-axis of the wrist assembly coordinate system 650 may coincide with the central axis 207 of the instrument holder 280. In some embodiments, one or more sensors may be used to measure the rotational position of the wrist assembly 270 relative to the forearm assembly 260, and this is further used to determine the coordinate transformation relationship 645 between the wrist assembly coordinate system 650 and the forearm assembly coordinate system 640. In some embodiments, based on the combination of transformation relationships 615-645, a kinematic model between wrist assembly coordinate system 650 and the base coordinate system 610 may be established, and the kinematic model may further be used to describe the motion information of the wrist assembly 270 in the base coordinate system 610. The motion information includes position information and posture information. In some embodiments, based on the motion information of the wrist assembly 270 in the base coordinate system 610 and the transformation relationships 615-645, through inverse kinematics, the motion information of the joints of the lifting column 240, upper arm assembly 250, forearm assembly 260, and wrist assembly 270 may be determined.

The kinematic model 600 further includes an instrument holder coordinate system 660 established at the end of the instrument holder 280. In some embodiments, coordinate system 660 may be established at the end of the instrument holder 280 and on a central axis 207, used to describe the motion information of the instrument holder 280. In some embodiments, one or more sensors may be used to measure the rotational position of the instrument holder 280 relative to the wrist assembly 270, and this is further used to determine the coordinate transformation relationship 655 between the instrument holder coordinate system 660 and the wrist assembly coordinate system 650. In some embodiments, based on the combination of transformation relationships 615-655, a kinematic model between coordinate systems 660 and 610 may be established, and the kinematic model may further be used to describe the motion information of the instrument holder 280 in the base coordinate system 610. The motion information includes position information and posture information. In some embodiments, based on the motion information of the instrument holder 280 in the base coordinate system 610 and the transformation relationships 615-655, through inverse kinematics, the motion information of the joints of the mechanical arm 200 may be determined, enabling accurate motion control of the endpoint of the mechanical arm 200 of the surgical robot 110.

The kinematic model 600 further includes a remote center of motion coordinate system 670 for the mechanical arm 200, whose origin coincides with a remote center of motion 160. In some embodiments, through the mechanical geometric relationships on the instrument holder 280, the coordinate transformation relationship 665 between the remote center of motion coordinate system 670 and the instrument holder coordinate system 660 may be determined. Furthermore, based on the combination of transformation relationships 615-665, the coordinate transformation relationship 675 between coordinate systems 670 and 610 may be established, and this may further be used to describe the positional motion information of the remote center of motion 160 of the mechanical arm 200 in the base coordinate system 610 of the chassis and the posture motion information of the instrument holder 280. In some embodiments, knowing the positional motion information of the remote center of motion 160 in the base coordinate system 610 of the chassis and the posture motion information of the instrument holder 280, based on the transformation relationship 675, through inverse kinematics, the motion information of the joints of the mechanical arm 200 may be determined, thereby enabling hand-guiding dragging translation and rotation of the mechanical arm 200 within the workspace.

Among them, when the control device 290 controls the instrument holder 280 to perform docking movement, it is configured to:
In response to a first docking instruction, control the mechanical arm 200 moves and allow the instrument holder 280 to move freely under external force, to rotate or translate the remote center of motion 160.

In response to a second docking instruction, control the mechanical arm 200 moves and maintain the remote center of motion 160 stationary relative to the chassis, and allow the instrument holder 280 to rotate around the remote center of motion 160 at a first speed under external force, and prohibit translational movement of the instrument holder 280 along three axes of a Cartesian coordinate system. The first speed is associated with the external force applied to the instrument holder 280.

In response to a third docking instruction, control the mechanical arm 200 moves and allow the instrument holder 280 to perform translational movement along the three axes of the Cartesian coordinate system at a second speed under the external force, and prohibit the instrument holder 280 from rotating around the remote center of motion 160. The second speed is associated with the external force applied to the instrument holder 280.

Among them, free movement may be used for an initial positioning of the instrument holder 280, for example, the instrument holder 280 is dragged from an initial retracted state (retracted position) to a surgical location (docking position) of the patient. For example, the surgical location is abdomen of the patient 130 when the patient 130 is doing a laparoscopic surgery. After the docking device 140 successfully docks with the cannula 150, the remote center of motion 160 is located on a central axis of the cannula 150 and flush with the incision 170 where the cannula 150 is inserted into the patient 130. Rotation around the remote center of motion 160 may be used to align the docking device 140 of the instrument holder 280 with the cannula 150. Translational movement may be used to translate the instrument holder 280 towards the cannula 150, generally performed after alignment, or for slight adjustments of distance up, down, left, or right.

Furthermore, the above docking movement may be pushed by manual or automatically executed by program control. When the docking movement is automatically executed by the program control, external force input is ineffective.

Furthermore, in response to a fifth docking instruction, the control device 290 controls the movement of the mechanical arm 200 to allow the instrument holder 280 to perform uniform translational movement along the three axes of the Cartesian coordinate system. External force input is ineffective at this time.

Furthermore, only one type of docking movement may be used alone, or they may be used comprehensively. The sequence of use in comprehensive use may be changed according to the doctor's clinical experience, and the usage method is not limited to the above embodiments but may be freely chosen by the operator according to the actual situation.

For example, in some cases, the medical staff may align docking device 140 with the cannula 150 using only free movement based on experience. In some cases, the medical staff need to execute free movement, rotation around the remote center of motion 160, and translational movement in sequence to align and connect docking device 140 with the cannula 150. In some cases, besides sequential execution, it may be necessary to switch back and forth between various docking movements to complete the docking.

Specifically, the external force applied by medical staff through manual pushing or pulling on the instrument holder 280 is detected by sensors arranged on the mechanical arm 200. When the sensor detects an external force, the sensor sends a detection result to the control device 290, enabling the control device 290 to control the instrument holder 280 to perform docking movement based on the external force.

Among them, the sensor may be a force sensor, or a torque sensor, or a force detection unit, or a torque detection unit.

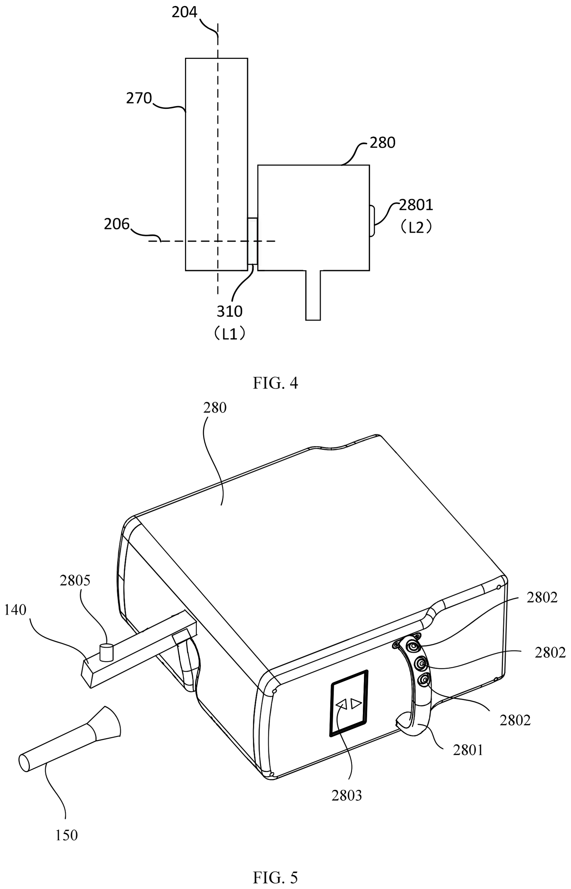

Furthermore, referring to Fig. 4, Fig. 4 is a simplified schematic diagram of the arrangement of sensor for detecting external force on the surgical robot 110. The sensor for detecting external force includes a first force sensor L1 and a second force sensor L2. The first force sensor L1 is arranged between the instrument holder 280 and the distal end of the mechanical arm 200, for example, the first force sensor L1 may be arranged on a pivot axis 206 of the instrument holder 280 near a side of the wrist assembly 270. The second force sensor L2 is arranged on the handle 2801 of the instrument holder 280. Other positions may also be considered for adding sensors to detect external force based on production cost and detection effectiveness, or only one sensor for detecting external force may be chosen. This is not limited here.

Furthermore, referring to Fig. 5, Fig. 5 is a schematic diagram of the structure of an embodiment of the instrument holder 280. As shown in Fig. 5, the instrument holder 280 includes at least one first input device 2802. The first input device 2802 is used to input one instruction among the first docking instruction, the second docking instruction and the third docking instruction.

Among them, the instrument holder 280 further includes a handle 2801. The handle 2801 is provided with a second force sensor L2. The second force sensor L2 is used to detect the amount of external force input applied to the handle 2801. The control device 290 is configured to transform the external force input amount through coordinate system transformations and inverse kinematics calculations, and output motion commands to the mechanical arm 200.

Compared to the first force sensor L1 arranged on the pivot axis 206, the second force sensor L2 installed on the handle 2801 may more accurately perceive the force applied by hand because:
A. The first force sensor L1 is located on the joint of the mechanical arm 200, and the joint carries the instrument holder 280. The instrument holder 280 usually has a large mass and a far center of gravity. The gravity of the instrument holder 280 loads the first force sensor L1, resulting in low acquisition accuracy of the hand dragging force by the first force sensor L1.
B. The force on the cannula 150 (connection between cannula 150 and patient 130) will be transmitted to the first force sensor L1. The first force sensor L1 may not distinguish whether the force is from hand dragging or from the cannula 150.
C. Adding the second force sensor L2 at the handle 2801, the force received by the second force sensor L2 comes directly from the hand and is not affected by the gravity of the instrument holder 280 or the force on the cannula 150.

In summary, setting the second force sensor L2 on the handle 2801 allows for more precise operation of the instrument holder 280 during preoperative cannula 150 docking and intraoperative cannula 150 adjustment, further reducing danger.

Among them, the first input device 2802 is arranged on the handle 2801. For example, the first input device 2802 may be designed as buttons. When the medical staff grab the handle 2801 to control and drag the instrument holder 280, they may choose to press different buttons while dragging. Compared to being placed in other locations, having the first input device 2802 on the handle 2801 is easier to operate. For example, it may be operated with one hand, and it is easier to switch the motion mode of the instrument holder's docking movement.

Among them, the instrument holder 280 further includes a docking device 140 and a second input device 2805. The docking device 140 is used to dock with the cannula 150. The docking device 140 is located at the distal end of the instrument holder 280. The second input device 2805 is located on the docking device 140. The second input device 2805 is used to input a fourth docking instruction.

Among them, a sensor is arranged between the instrument holder 280 and the distal end of the mechanical arm 200. The sensor is used to detect the amount of external force input applied to the docking device 140. The control device 290 is configured to transform the external force input amount through coordinate system transformations and inverse kinematics calculations, and output motion commands to the mechanical arm 200. Specifically referring to Fig. 4, the sensor is the first force sensor L1, arranged between the instrument holder 280 and the distal end of the mechanical arm 200, for example, the first force sensor L1 may be arranged on the pivot axis 206 of the instrument holder 280 near the side of the wrist assembly 270.

Furthermore, in response to a fourth docking instruction, the control device controls the mechanical arm to move and allows the instrument holder to perform translational movement along the three axes of the Cartesian coordinate system at a third speed under external force, and prohibits the instrument holder from rotating around the remote center of motion. The third speed is associated with the external force applied to the instrument holder. Under substantially the same external force applied to the instrument holder, the third speed is less than the second speed.

The external force at this time is directly applied to the docking device 140, meaning medical staff directly grab the docking device 140 and press the second input device 2805 on it, thereby dragging the instrument holder 280 for translational movement. At this time, since a part grabbed by hand is the part that will contact the cannula 150, compared to grabbing the handle 2801 and dragging, it enables the cannula 150 docking process more direct. By reducing the speed of translational movement (the third speed is less than the second speed), the translation docking process may be controlled more finely.

Furthermore, in response to a docking success signal for the cannula or a lock instruction for the cannula, the control device 290 no longer responds to the first docking instruction and the fourth docking instruction.

Among them, the docking success signal is generated after the docking device 140 docks with the cannula 150. At this time, the instrument holder 280 is connected to the cannula 150 inserted into the human body through the docking device 140. If the movement is too free, there is a risk of harming the human body. Therefore, a range of docking movement must be reduced, and the function defined by the first docking instruction for free movement is set to invalid and the fourth docking instruction for direct docking movement is are set to invalid, only retaining the rotation around the remote center of motion 160 defined by the second docking instruction and the translational movement defined by the third docking instruction.

Furthermore, in response to the docking success signal and the second docking instruction, the control device 290 controls the instrument holder 280 to rotate around the remote center of motion 160 at a fourth speed under the external force. The fourth speed is associated with the external force applied to the instrument holder 280. Under substantially the same external force applied to the instrument holder 280, the fourth speed is less than the first speed.

Furthermore, in response to the docking success signal and the third docking instruction, the control device 290 controls the instrument holder 280 to perform translational movement along the three axes of the Cartesian coordinate system at a fifth speed under the external force. Among them, under substantially the same external force applied to the instrument holder 280, the fifth speed is less than the second speed.

Among them, after the docking device 140 is docked with the cannula 150, the speed of the docking movement of the instrument holder 280 with the surgical instrument installed should be set lower than the speed before installation. This is because the surgical instrument is already inserted into the human body through the cannula 150 at this time, and even slight movement may risk injury to the human body. Therefore, the range of docking movement must be further reduced, and the speed of docking movement must be lowered (the fourth speed is less than the first speed, the fifth speed is less than the second speed).

Among them, the docking success signal for the docking between the docking device 140 and the cannula 150 may be generated by manual triggering or automatically sensed by the instrument holder 280.

Among them, the coordinate system 660 of the instrument holder 280 may include multiple coordinate origins, further subdivided into multiple coordinate systems. Referring to Figs. 4 and 5, a first origin of the first coordinate system is the joint 310 connecting the instrument holder 280 to the mechanical arm 200. A second origin of the second coordinate system is the clamping point of the docking device 140 on the instrument holder 280. A third origin of the third coordinate system is the remote center of motion 160. This design allows the instrument holder 280 to use different coordinate systems for docking movements in different scenarios, further improving precision.

Among them, when the docking movement is free movement, the instrument holder 280 is limited to translating along the first coordinate system or second coordinate system and rotating around the first or second origin. Therefore, 5-DOF dragging may be performed, including three translations and two rotations. At this time, the first force sensor L1 is used for detecting the external force.

Among them, when the docking movement is translational movement, the instrument holder 280 is limited to translating along the second coordinate system or the third coordinate system. At this time, the second force sensor L2 is used for detecting the external force.

Among them, when the docking movement is the direct docking translational movement corresponding to the fourth docking instruction, the instrument holder 280 is limited to translating along the second coordinate system or the third coordinate system. At this time, the first force sensor L1 is used for detecting the external force.

Through the above subdivided coordinate systems and force sensor settings, the precision of operation may be further improved.

Furthermore, in response to a feed instruction, the control device 290 controls the instrument holder 280 to move along the direction of the central axis 180 of the cannula.

Among them, after the docking device 140 is docked with the cannula 150, the control device 290, in response to the feed instruction, causes the instrument holder 280 to perform automatic translational movement along the central axis 180 direction of the cannula 150. This is due to individual differences among patients 130 and the need for angle adjustment during surgery, requiring slight adjustments of the remote center of motion 160 preoperatively or intraoperatively.

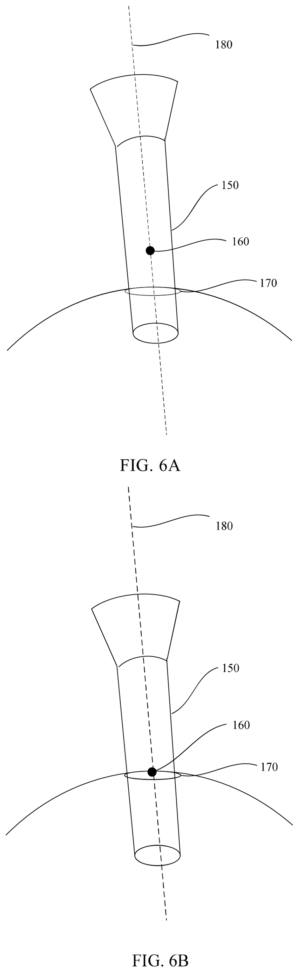

Specifically, referring to Fig. 6A, Fig. 6A is a schematic diagram of a scenario of an embodiment of the cannula 150 inserted into a human body. As shown in Fig. 6A, first, after the cannula 150 is connected to the instrument holder 280, when the instrument holder 280 performs fixed-point rotation around the remote center of motion 160, the cannula 150 is also pulled to perform fixed-point rotation around the remote center of motion 160. The cannula 150 is inserted into the human body. Only when the position of the remote center of motion 160 exactly coincides with the human body incision 170 will the fixed-point rotation not cause any additional damage to the patient's incision 170. This is because if the position of the remote center of motion 160 is above the human body incision 170 and is outside the body, it will cause the part of the cannula 150 below the remote center of motion 160 to have an excessively large rotation angle during fixed-point rotation, thereby pulling the wound (as shown in Fig. 6A). If the position of the remote center of motion 160 is below the human body incision 170 and is inside the body (not shown), it will cause the part of the cannula 150 above the remote center of motion 160 to have an excessively large rotation angle during fixed-point rotation, thereby pulling the wound.

For the above reasons, when the lead surgeon inserts the cannula 150 into the human body, the insertion depth must be just enough to enable the remote center of motion 160 coincide with the human body incision 170 (as shown in Fig. 6B), thus avoiding any additional damage to the patient's incision 170.

Among them, the cannula 150 is provided with an initial mark for indicating the remote center of motion 160.

An initial mark may be added to the outside of the cannula 150 to indicate the position of the remote center of motion 160, for example, a red dot, to facilitate the doctor's operation.

Furthermore, as shown in Fig. 6A, the fixation between the cannula 150 and the human incision 170 relies only on the friction of the patient's 130 skin tissue and is not completely reliable. It is possible that after successful docking, the remote center of motion 160 of the instrument holder 280 and the human incision 170 become misaligned.

Therefore, the medical staff are allowed to use the feed instruction to control the movement of the instrument holder 280 to perform automatic translational movement with a single degree of freedom along the central axis 180 direction of the cannula 150, thereby adjusting the insertion depth of the cannula 150 into the human body, ultimately ensuring that the remote center of motion 160 coincides with the human incision 170.

Among them, referring to Fig. 5, the instrument holder 280 further includes a third input device 2803. The third input device 2803 is used to input the feed instruction. To further ensure the precision of the docking movement, in the aforementioned automatic translational movement, the movement speed will not be controlled by the force on the handle 2801 but will be uniform automatic translation at a preset speed. The third input device 2803 may be a touch screen, including multiple icon buttons. For example, holding down the forward icon button on the touch screen will cause the instrument holder 280 to perform forward automatic translational movement along the central axis 180 direction of the cannula 150, ultimately causing the cannula 150 to move inward into the body. Holding down the backward icon button on the touch screen will cause the instrument holder 280 to perform backward automatic translational movement along the central axis 180 direction of the cannula 150, ultimately causing the cannula 150 to move outward from the body.

Furthermore, in response to a reconfiguration instruction, a distance from the remote center of motion 160 to the instrument holder 280 along the direction of the central axis 207 of the instrument holder 280 is reconfigured. The control device 290 controls the instrument holder 280 to rotate around the reconfigured remote center of motion 160.

Among them, the third input device 2803 is also used to input the reconfiguration instruction. The control device 290 corrects position of the remote center of motion 160 according to the reconfiguration instruction. This is because, preoperatively or intraoperatively, after the surgical instrument is inserted into the human body through the cannula 150, further adjustment to the position of the cannula 150 and the remote center of motion 160 is needed based on individual patient 130 body type differences and the condition of lesion H. At this time, the central axis 207 of the instrument holder 280 substantially coincides with the central axis 180 of the cannula 150. Therefore, correction may be done by reconfiguring the distance from the remote center of motion 160 to the instrument holder 280 along the direction of the central axis 207 of the instrument holder 280.

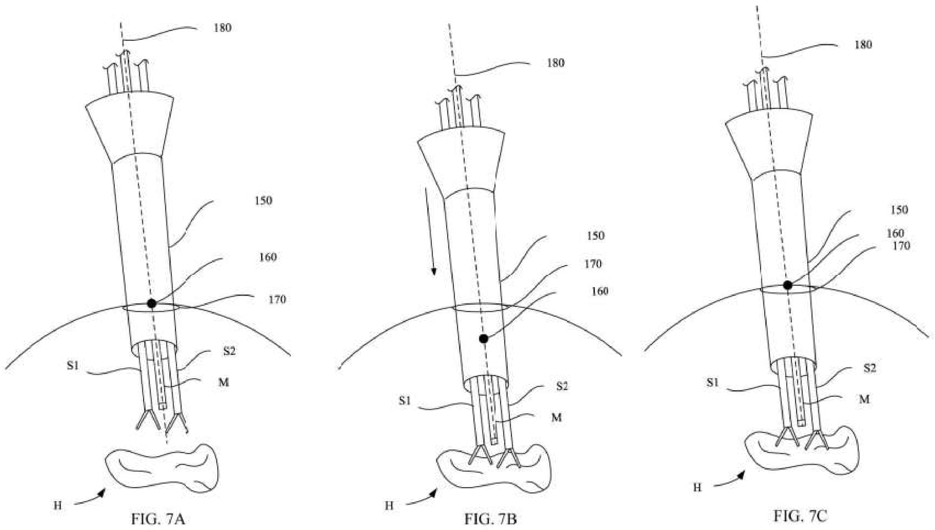

Specifically, refer to Figs. 7A-7C, which are schematic diagrams of a scenario of an embodiment of a surgical instrument inserted into a human body. As shown in Fig. 7A, M is an endoscope, S1 and S2 are surgical instruments, and H is a lesion inside the human body. It can be seen from Fig. 7A that because the lesion H is deep, the two surgical instruments (S1 and S2) have reached lowest boundary of the workspace but still cannot reach the target lesion H. At this time, the fourth docking instruction is used to control the instrument holder 280 and the cannula 150 to move forward as a whole, i.e., move the cannula 150 along the central axis 180, so that the surgical instruments S1, S2 may move forward to reach the target lesion H (as shown in Fig. 7B). But because the instrument holder 280 and cannula 150 have moved as a whole, the remote center of motion 160 no longer coincides with the human incision 170, posing a risk of human injury when the cannula 150 and instruments rotate around the remote center of motion 160. However, the cannula 150 position cannot be moved further back at this time, otherwise the surgical instruments S1, S2 would not reach the lesion H. Therefore, the remote center of motion 160 of the instrument holder 280 needs to be recalibrated, moving the remote center of motion 160 backward by the same distance to coincide with the human incision 170 (as shown in Fig. 7C).

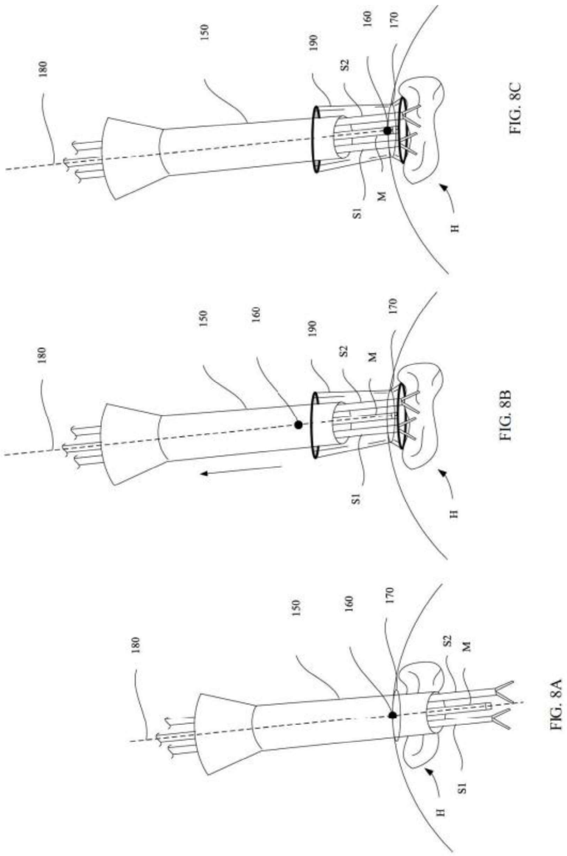

Similarly, referring to Figs. 8A-8C. Fig. 8A is a schematic diagram of a scenario of an embodiment of a surgical instrument inserted into a human body. As shown in Fig. 8A, because the lesion H is shallow (close to the patient's skin), the two surgical instruments S1, S2 have reached the highest boundary of the workspace but still cannot reach the target lesion H. At this time, the fourth docking instruction is used to control the instrument holder 280 and the cannula 150 to move backward as a whole, i.e., move the cannula 150 along the central axis 180, so that the instruments may move backward to reach the target lesion H (as shown in Fig. 8B). But because the mechanical arm 200 and cannula 150 have moved as a whole, the remote center of motion 160 no longer coincides with the human incision 170, posing a risk of human injury when the cannula 150 and instruments rotate around the remote center of motion 160. The cannula 150 position cannot be moved further down at this time, otherwise the surgical instruments would not reach the lesion H. Therefore, the remote center of motion 160 of the instrument holder 280 needs to be recalibrated, moving the remote center of motion 160 forward by the same distance to coincide with the human incision 170 (as shown in Fig. 8C).

Among them, as shown in Figs. 8B and 8C, the end of the cannula 150 is provided with an incision protector 190. The incision protector 190 envelops the end of the cannula 150 and is inserted into the human body through the human incision 170. The remote center of motion 160 is located on the incision protector 190. This is because there may also be a situation where the entire cannula 150 is removed from the human body. At this time, an incision protector 190 needs to be added. The incision protector 190 may protect the incision 170, prevent the human incision 170 from being scratched by surgical instruments S1, S2, and prevent intra-abdominal gas leakage.

Among them, the reconfiguration instruction includes a first parameter and a second parameter. The first parameter is correction direction of the remote center of motion 160, including forward and backward. The second parameter is correction distance data of the remote center of motion 160.

Among them, the cannula 150 is provided with scales. Furthermore, scales are provided near the initial mark indicating the remote center of motion 160. This allows accurate translation distance data to be read when adjusting the distance of the remote center of motion 160. For example, if the cannula 150 moves forward by 5mm, then during remote center of motion 160 correction, input backward 5, i.e., the first parameter is backward, and the second parameter is 5.

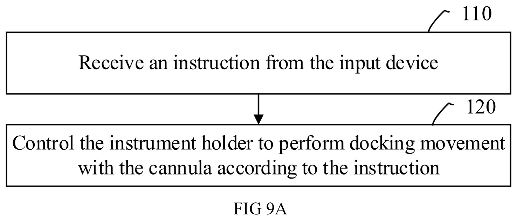

On the other hand, referring to Fig. 9A, the present disclosure provides a cannula docking control method for the above surgical robot. The surgical robot includes a mechanical arm, and the end of the mechanical arm is provided with an instrument holder for installing the surgical instruments. The method includes:
110: Receive an instruction from the input device.
120: Control the instrument holder to perform docking movement with the cannula according to the instruction.

For other structural descriptions of the surgical robot, please refer to the above embodiments and will not be repeated here.

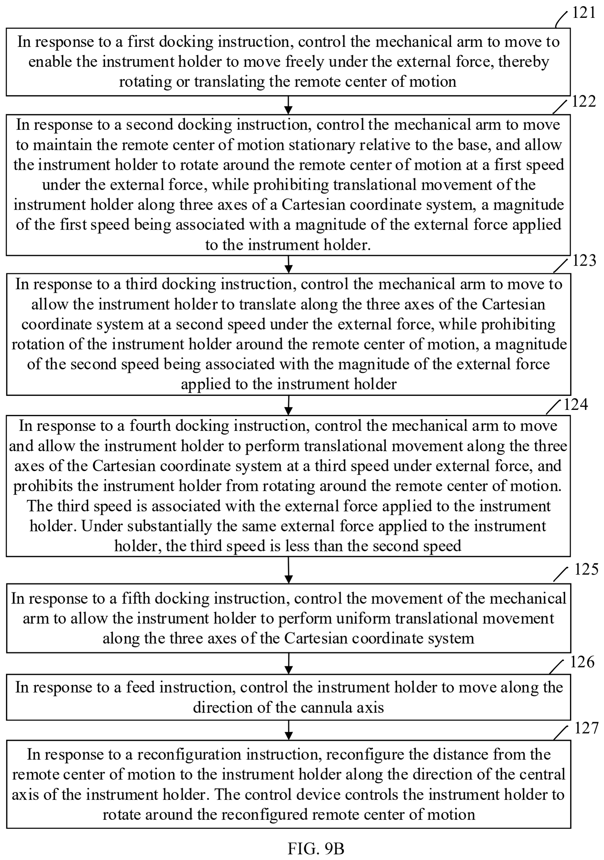

Furthermore, referring to Fig. 9B, for different instructions, the cannula docking control method includes different docking movements:
121: In response to a first docking instruction, control the mechanical arm to move to enable the instrument holder to move freely under the external force, thereby rotating or translating the remote center of motion.
122: In response to a second docking instruction, control the mechanical arm to move to maintain the remote center of motion stationary relative to the base, and allow the instrument holder to rotate around the remote center of motion at a first speed under the external force, while prohibiting translational movement of the instrument holder along three axes of a Cartesian coordinate system, a magnitude of the first speed being associated with a magnitude of the external force applied to the instrument holder.
123: In response to a third docking instruction, control the mechanical arm to move to allow the instrument holder to translate along the three axes of the Cartesian coordinate system at a second speed under the external force, while prohibiting rotation of the instrument holder around the remote center of motion, a magnitude of the second speed being associated with the magnitude of the external force applied to the instrument holder.

Furthermore, the method also includes the step:
124: In response to a fourth docking instruction, control the mechanical arm to move and allow the instrument holder to perform translational movement along the three axes of the Cartesian coordinate system at a third speed under external force, and prohibits the instrument holder from rotating around the remote center of motion. The third speed is associated with the external force applied to the instrument holder. Under substantially the same external force applied to the instrument holder, the third speed is less than the second speed.

Furthermore, the method also includes the step:
125: In response to a fifth docking instruction, control the movement of the mechanical arm to allow the instrument holder to perform uniform translational movement along the three axes of the Cartesian coordinate system.

Furthermore, the method also includes the step:
In response to in response to a docking success signal for the cannula or a lock instruction for the cannula, no longer respond to the first docking instruction and the fourth docking instruction.

Furthermore, the method also includes the step:
In response to the docking success signal and the second docking instruction, control the instrument holder to rotate around the remote center of motion at a fourth speed under the external force. The fourth speed is associated with the external force applied to the instrument holder. Under substantially the same external force applied to the instrument holder 280, the fourth speed is less than the first speed.

Furthermore, the method also includes the step:
In response to the docking success signal and the third docking instruction, control the instrument holder to perform translational movement along the three axes of the Cartesian coordinate system at a fifth speed under the external force. Under substantially the same external force applied to the instrument holder, the fifth speed is less than the second speed.

Furthermore, the method also includes the step:
126: In response to a feed instruction, control the instrument holder to move along the direction of the cannula axis.

Furthermore, the method also includes the step:
127: In response to a reconfiguration instruction, reconfigure the distance from the remote center of motion to the instrument holder along the direction of the central axis of the instrument holder. The control device controls the instrument holder to rotate around the reconfigured remote center of motion.

Furthermore, the method also includes the step:
In response to a sixth docking instruction, control the instrument holder to automatically execute the various docking movements described above.

The execution of the above instructions may be automatic and sequential, or the user may manually choose to execute only one or a combination of several.

For example, a sequence for automatic execution is:
S10: Execute the first docking instruction.
S20: Determine if docking is successful. If the result is No, proceed to step S30; if the result is Yes, end the process.
S30: Execute the second docking instruction.
S40: Determine if docking is successful. If the result is No, proceed to step S50; if the result is Yes, end the process.
S50: Execute the third docking instruction.
S60: Determine if docking is successful. If the result is No, proceed to step S30; if the result is Yes, end the process.

The execution time for steps S10, S130, S150 may be pre-configured. For example, proceed to step S20 30 seconds after executing the first docking instruction.

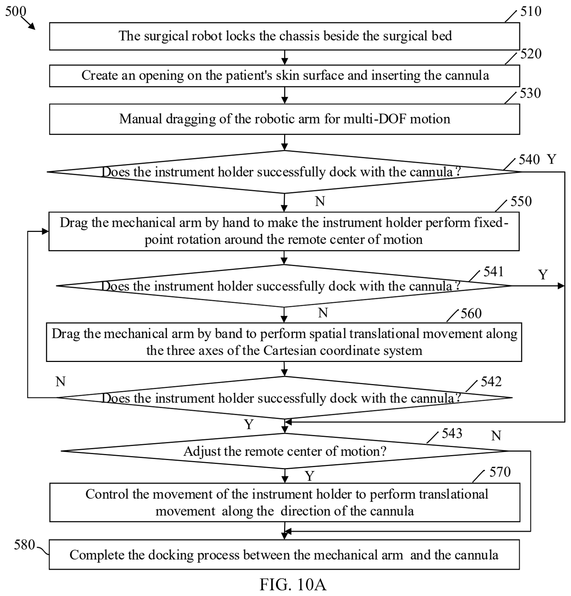

Specifically, referring to Fig. 10A, Fig. 10A is a flowchart of an embodiment of cannula 150 docking.

As shown, after the medical staff push the surgical robot 110 close to the surgical bed 120 to a target position that is expected, a locking process 510 for the chassis is executed. Considering that a base reference coordinate system for motion calculation of the mechanical arm 200 components is usually established on the chassis, locking the chassis 210 ensures that the base reference coordinate system for each mechanical motion joint remains stationary relative to the ground. Moreover, surgery requires the remote center of motion 160 of the surgical robot 110 to remain stationary relative to the ground. Therefore, locking the chassis before surgery effectively avoids surgical risks caused by non-subjective movement of the chassis during surgery. After completing a sterile environment setup for the patient 130, the lead surgeon typically comprehensively considers the patient's 130 surgical location and the operational fluency of the surgical robot 110, finally determining the surgical incision 170 on the patient's 130 skin surface, and executes the process 520 of creating an opening on the patient's 130 skin surface and inserting the cannula 150. This process places the remote center of motion 160 on the central axis 180 of the cannula 150 and coincident with the patient's 130 body incision 170. Before process 520, the instrument holder 280 of the robot is usually initially positioned away from the cannula 150 of the patient 130 to release as much operating space as possible for the docking between the cannula 150 and the patient 130. After completing the docking operation between the cannula 150 and the patient 130, the medical staff will execute the process 530 of dragging the mechanical arm 200 with multiple degrees of freedom by hand, allowing the instrument holder 280 to quickly approach the cannula 150 at the human incision 170 and begin docking with it. During process 540, if the instrument holder 280 does not successfully dock with the cannula 150 during dragging. Unsuccessful docking situations include but is not limited to, for example, failure to dock accurately in one attempt, or slow successful docking after frequent repeated operations of the instrument holder 280 movement, or subjective unwillingness to perform the docking operation with the cannula 150. The remote center of motion 160 on the instrument holder 280 does not coincide with the human incision 170, and the central axis of the surgical instrument on the instrument holder 280 does not coincide with the central axis 180 of the cannula 150 already inserted into the patient's 130 body. Therefore, process 550 will continue: dragging the mechanical arm 200 by hand to enbale the instrument holder 280 perform fixed-point rotation around the remote center of motion 160. When the rotatable range of the cannula 150 around the patient's 130 body incision 170 is not at the boundary limit or is near the center of the workspace, the main goal of process 550 is to prioritize adjusting the posture of the instrument holder 280 in space until the central axis of the surgical instrument coincides with the central axis 180 of the cannula 150 already inserted into the patient's 130 body. Based on this, after executing process 541 to determine if the instrument holder 280 is successfully docked with the cannula 150, if the instrument holder 280 is not successfully docked during dragging, the unsuccessful docking situations in process 540 also apply here, while keeping the posture of the instrument holder 280 relative to the chassis base coordinate system unchanged after process 550, continue to execute process 560: dragging the mechanical arm 200 by band to perform spatial translational movement along the three axes of the Cartesian coordinate system, approaching the cannula 150 for docking. If docking is unsuccessful, process 550 may be executed again to adjust the posture of the instrument holder 280. Repeated execution of processes 550 and 560 may achieve successful docking between the instrument holder 280 and the cannula 150. If the result of process 540, 541, or 542 is successful docking, the medical staff may selectively execute process 543 based on the quality of the docking between the instrument holder 280 and the cannula 150. Considering that the fixation between the cannula 150 and the human incision 170 relies only on the friction of the patient's 130 skin tissue and is not completely reliable, it is possible that after successful docking in process 542, the remote center of motion 160 of the instrument holder 280 and the human incision 170 become misaligned. Therefore, the medical staff are allowed to continue executing process 570: controlling the movement of the instrument holder 280 to perform translational movement with a single degree of freedom along the central axis 180 direction of the cannula 150. The amount of translational movement is determined by the medical staff based on an accurate assessment of the actual docking quality on site. Typically, this movement is tiny to avoid causing additional damage to the human incision 170 due to excessive movement while achieving coincidence between the remote center of motion 160 and the human incision 170. This finally completes the docking process 580 between the mechanical arm 200 of the surgical robot 110 and the cannula 150, laying the foundation for the subsequent installation and use of the surgical instruments.

Furthermore, the above embodiment involves manual dragging of the instrument holder 280 for docking. In other embodiments, it can also be automatic docking, for example, automatically switching to step 550 if not successfully docked within a certain range after moving in step 530, and automatically switching to step 560 if not successfully docked and moved within a certain range in step 550.

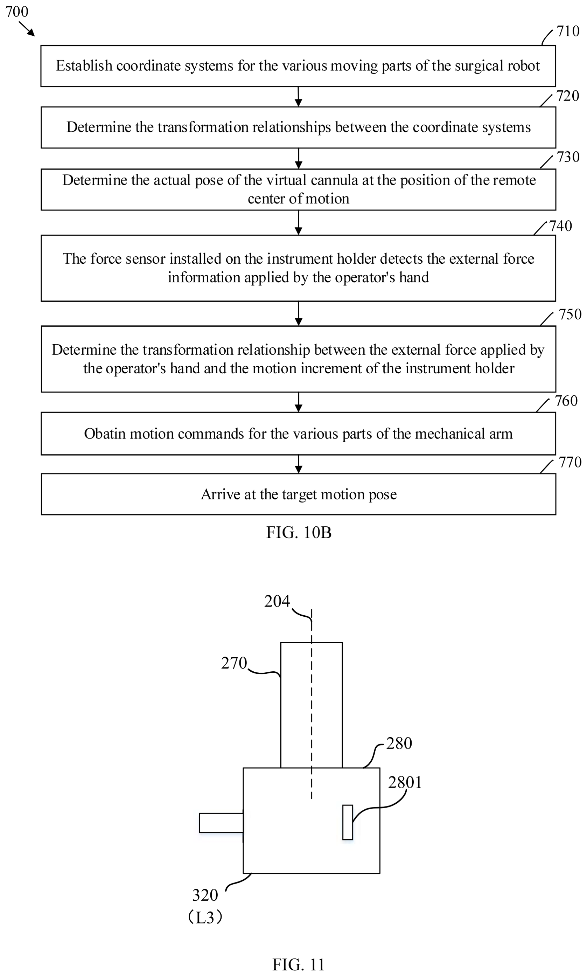

Furthermore, referring to Fig. 10B, Fig. 10B is a simplified flowchart of a control method 700 for dragging the mechanical arm 200 to move by hand.

As shown in Fig. 10B, first, based on kinematic coordinate system establishment rules, complete the process 710 of establishing coordinate systems for the various moving parts of the surgical robot 110. Then execute process 720: determine the transformation relationships between the coordinate systems based on kinematic parameter information. Based on the transformation relationships between the coordinate systems, further execute process 730: determine the actual pose of the virtual cannula 150 at the position of the remote center of motion 160. After the cannula 150 completes docking with the patient's 130 body incision 170, use the pose information of the cannula 150 as the target motion pose for the end of the mechanical arm 200. When the operator's hand applies an external force to the instrument holder 280, execute process 740: the force sensor installed on the instrument holder 280 detects the external force information applied by the operator's hand. In process 750, perform data filtering on the external force information detected by the force sensor to eliminate environmental interference, and further apply mathematical modeling methods (such as motion calibration or motion fitting) to determine the transformation relationship between the external force applied by the operator's hand and the motion increment of the instrument holder 280. In process 760, using the external force applied by the operator's hand as the input of the system, after several coordinate transformations and kinematic calculations, motion commands for the various parts of the mechanical arm 200 may be obtained. The drive units of the moving parts (such as motor and driver combinations) execute motion control according to the received control commands. Movement stops after process 770 is completed and the target motion pose is arrived, completing the process of hand-guiding dragging the mechanical arm 200 motion control.

Furthermore, the inventors found that before, during, and after surgery, situations exist where the instrument holder 280 squeezes the patient, including:
On one hand, after the mechanical arm 200 is docked with the cannula 160 and before the surgical instruments are installed, mis-operation by the medical staff may cause the instrument holder 280 at the end of the mechanical arm 200 to squeeze the patient's skin, causing secondary injury to the patient.

On the other hand, after the instruments are installed and the lead surgeon begins the surgery, since the lead surgeon operates based on the image view from the master console, which shows the surgical field inside the patient, the lead surgeon cannot see actual movement of the mechanical arm 200 outside the patient. The movable workspace of the mechanical arm 200 of the surgical robot 110 is much larger than the workspace required for actual surgical operation. Therefore, without any external feedback, there is a risk of the mechanical arm 200 contacting or even squeezing the patient during the lead surgeon's operation of the surgical robot 110, which will cause secondary injury to the patient.

Based on this, referring to Figs. 1 and 2, an embodiment of the present disclosure also provides a surgical robot 110, comprising: a mechanical arm 200 and a control device 290, wherein the end of the mechanical arm 200 is provided with an instrument holder 280 for installing the surgical instruments, the control device 290 is configured to control the movement of the mechanical arm 200 so that multiple surgical instruments rotate around the same remote center of motion 160, and:
in response to a boundary setting instruction, set the current posture of the instrument holder 280 as the motion boundary for the instrument holder's 280 movement around the remote center of motion 160.
in response to the instrument holder 280 rotating around the remote center of motion 160 to the motion boundary, control the instrument holder 280 to move in a preset motion mode.

The structure of the surgical robot 110 in this embodiment refers to the foregoing various embodiments and will not be repeated here.

Among them, the motion boundary is a physical position. When the instrument holder 280 reaches the position of the motion boundary during movement, it is not allowed to continue moving in the current direction. For example, if the motion boundary is set where the bottom of the instrument holder 280 is 5cm from the patient's abdomen, then when the instrument holder 280 moves downward to that boundary position, it cannot continue downward, i.e., the distance from the bottom of the instrument holder 280 to the patient's abdomen cannot be less than 5cm, thus preventing squeezing of the patient's abdomen.

When the anti-squeezing state is enabled, and it is detected that the instrument holder 280 has reached the motion boundary, control the instrument holder 280 to move in a preset motion mode. For example, it may only rotate but may not translate downward, so the distance between the instrument holder 280 and the patient will not decrease further.

Among them, moving in the preset motion mode includes:
moving along other directions different from the current motion direction, or ceasing movement.

For example, it may translate left or right but not down, so the distance to the patient does not decrease. Or move in the direction opposite to the current motion; if the current motion direction is moving down, change to moving up, so the distance to the patient increases.

When movement reaches the motion boundary, the surgical robot 110 issues a warning visually (e.g., an indicator light) or audibly (e.g., voice). This allows the operator to immediately respond and adjust the movement mode of the instrument holder 280 to prevent squeezing the patient.

In response to a cancel anti-squeezing instruction, set the motion boundary to the initial motion boundary. Among them, the initial motion boundary includes no boundary.

Among them, the input of the above boundary setting instruction is done through an input device provided on the instrument holder 280. The input device includes a mechanical button and/or a touch screen, and may further include other remote, and the boundary setting instruction is input by the remote.

With the solution of this embodiment, the operator may activate the setting of the motion boundary before, during, and after surgery according to the actual situation. Subsequently, the control device 290 controls the instrument holder to move within the range not exceeding the motion boundary, thereby preventing the instrument holder from squeezing the patient. When anti-squeezing operation is not needed, the function may be turned off, allowing the instrument holder to enter the target state more freely and quickly. Furthermore, visual or audible warnings are issued when reaching the free boundary, enabling the operator to timely adjust the current movement direction and mode. Furthermore, a preset initial motion boundary may be set, automatically activated upon startup. When the anti-squeezing function is turned off, the motion boundary is automatically restored to the initial motion boundary. Presetting may further prevent situations where the operator forgets to set the motion boundary, improving operational efficiency.

Among them, the control device 290 is further configured to:
In response to a reconfiguration instruction, reconfigure the distance from the remote center of motion 160 to the instrument holder 280 along the direction of the central axis 207 of the instrument holder 280. The instrument holder 280 rotates around a reconfigured remote center of motion 160.

As described in the previous embodiment, when the cannula 160 moves relative to the incision, the reconfiguration instruction should be used to correct the position of the remote center of motion 160, so that the instrument holder 280 rotates around the reconfigured remote center of motion 160. Accordingly, the range of the motion boundary will also change. The mapping relationship between the change in motion boundary and the change in remote center of motion is set as: y=f(x), where y is a variation of the motion boundary, x is a variation of the distance from the remote center of motion 160 to the instrument holder 280, and f is a pre-stored mapping relationship. For example, f may be preset based on experience; how much x changes determines how much the corresponding motion boundary y must change to avoid squeezing the patient (within a safe range).

Among them, the control device 290 is further configured to: in response to the distance from the remote center of motion 160 to the instrument holder 280 being configured to decrease, cause the motion boundary to correspondingly decrease to within a safe range; in response to the distance from the remote center of motion 160 to the instrument holder 280 being configured to increase, cause the motion boundary to correspondingly increase to within a safe range.

Among them, the control device 290 is further configured to: in response to the reconfiguration of the distance from the remote center of motion 160 to the instrument holder 280, cause the motion boundary to become invalid. This may remind the user to reset the motion boundary.

Among them, reconfiguring the distance from the remote center of motion 160 to the instrument holder 280 includes modifying a transformation relationship from the coordinate system of the remote center of motion 160 to the base coordinate system.

Among them, modifying the transformation relationship from the coordinate system of the remote center of motion 160 to the base coordinate system includes: translating the coordinate system of the remote center of motion 160 along the central axis 207 of the instrument holder.

Among them, the control device 290 is further configured to: in response to translation of the instrument holder 280 or rotation not around the remote center of motion 160, adjust the mechanical arm 200 to maintain the position of the remote center of motion 160 relative to the base coordinate system unchanged.

Among them, the surgical robot 110 further includes an identification device (not shown) for identifying the position of the human body incision 170. The distance from the remote center of motion 160 to the instrument holder 200 is relatively fixed and configurable. The control device 290 is configured to: control the instrument holder 280 moves and enable an origin of a coordinate system of the remote center of motion 160 coincide with an origin of a coordinate system based on the incision 170. The coordinate system based on the incision 170 is a coordinate system established by the control device 290 based on the human body incision 170 position identified by the identification device.

Coordinate system transformations are as described in the previous embodiments and will not be repeated here.

Among them, the mechanical arm 200 is provided with a sensor. The sensor is configured to detect squeezing when the mechanical arm 200 contacts the patient. The control device 290 is further configured to, in response to a signal from the sensor, control the mechanical arm 200 to perform anti-squeezing processing. The anti-squeezing processing includes: in response to the mechanical arm 200 squeezing the patient, control the instrument holder 280 to move in a preset motion mode.

Among them, the sensor includes a first sensor. The first sensor is arranged at the joint between the instrument holder 280 and the mechanical arm 200. The sensor is a non-contact force sensor.

Among them, the sensor includes a second sensor. The second sensor is arranged on the bottom surface of the instrument holder 280. The sensor is a contact force sensor.

The sensor for detecting the external force may be arranged at one or several positions on the instrument holder 280. Furthermore, the sensor may be a one-dimensional force sensor or a multi-dimensional force sensor. In some embodiments, as shown in Fig. 4, the first sensor is arranged at a connection 310 between the instrument holder 280 and the wrist assembly 270 (the first force sensor L1). Under the external force, a slight relative motion will occur between the instrument holder 280 and the wrist assembly 270, and the sensor detects the external force during a conversion of mechanical motion to electrical signal.

In some embodiments, as shown in Fig. 11, the second sensor is allowed to be arranged at the bottom 320 of the instrument holder 280 (the third force sensor L3). The sensor may be a contact force sensor, including but not limited to types such as a resistive force sensor, a capacitive force sensor, a piezoelectric force sensor, etc.

When the external force is greater than a first threshold that is preset, a squeeze warning instruction is generated.

When the external force is less than a second threshold that is preset, a warning release instruction is generated.

Among them, the first threshold represents the minimum value of a jump interval for the force sensor input. The external force is greater than the minimum value indicates a collision or squeeze exists at a location of the sensor. The second threshold represents the maximum value of a normal range for the force sensor input. The external force is less than maximum value indicates that the collision or squeeze at the location of the sensor has disappeared and it is within a safe position range.

Among them, moving in the preset motion mode includes: moving along other directions different from the current motion direction, or ceasing movement.

Among them, it further includes a cannula coupled to the instrument holder. The reconfigured remote center of motion is located on the axis of the cannula and outside the cannula.

Among them, it further includes a flexible incision protector. The proximal end of the incision protector is used to connect to the cannula. The distal end of the incision protector is used to connect to the human body incision. The remote center of motion is located within the incision protector.

The incision protector may further prevent squeezing, as explained in the above embodiments, and will not be repeated here.

With the solution of this embodiment, combined with a clever arrangement of the force sensor on the instrument holder, automatic detection of squeezing and corresponding automatic adjustment of movement may be achieved, enabling intelligent anti-squeezing operation. This solution does not require manual participation, reduces errors caused by manual operation, improves efficiency, and brings convenience to the operator while protecting the patient.

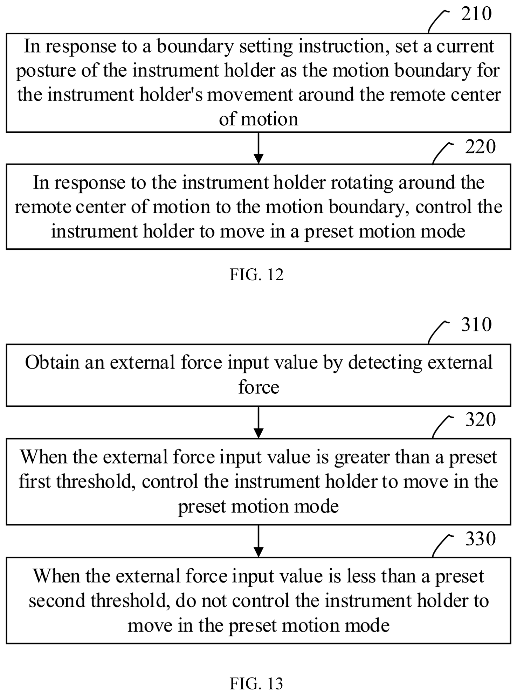

In yet another aspect, referring to Fig. 12, the present disclosure provides an anti-squeezing control method for the mechanical arm of the above surgical robot, including:
210: In response to a boundary setting instruction, set a current posture of the instrument holder as the motion boundary for the instrument holder's movement around the remote center of motion.
220: In response to the instrument holder rotating around the remote center of motion to the motion boundary, control the instrument holder to move in a preset motion mode.

Furthermore, it also includes: in response to a cancel anti-squeezing instruction, set the anti-squeezing to off and set the motion boundary to the initial motion boundary.

Among them, moving in the preset motion mode includes:
moving along other directions different from the current motion direction, or ceasing movement.

Furthermore, when the instrument holder reaches the motion boundary, control the surgical robot to issue a warning visually (e.g., indicator light) or audibly (e.g., voice).

Furthermore, it also includes:
310: Obtain an external force input value by detecting external force.
320: When the external force input value is greater than a preset first threshold, control the instrument holder to move in the preset motion mode.
330: When the external force input value is less than a preset second threshold, do not control the instrument holder to move in the preset motion mode.

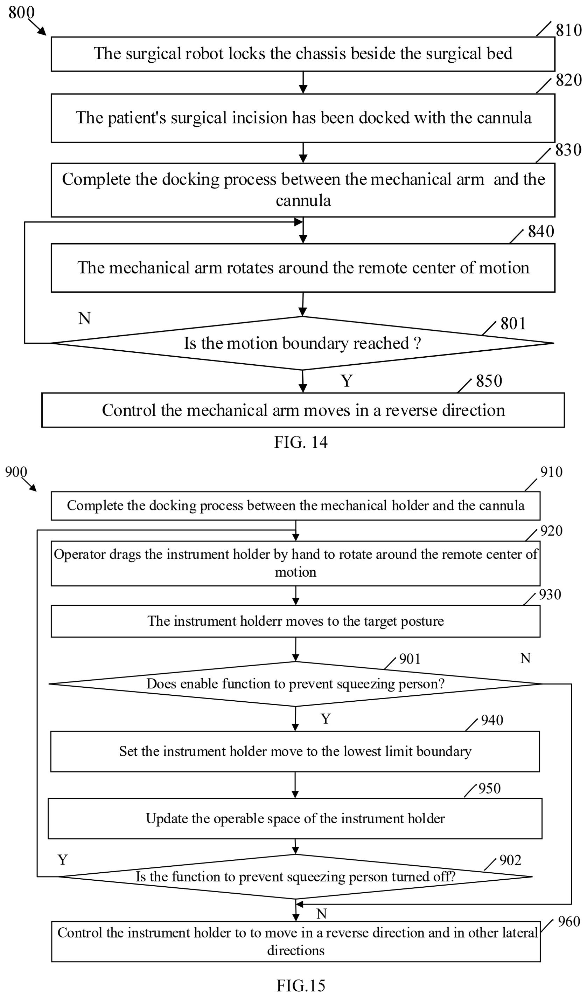

Specifically, referring to Fig. 14, Fig. 14 is a flowchart of an embodiment of motion control for the mechanical arm 200 of the surgical robot 110. The specific description of steps 810-840 please refer to the above various embodiments. In this embodiment, during the execution of the process 840 of the instrument holder 280 rotating around the remote center of motion, process 801 is concurrently executed to detect in real-time whether the instrument holder 280 reaches the motion boundary during movement. If the motion boundary is reached, execute process 850: the instrument holder 280 cannot continue moving in that direction but may continue moving in a reverse direction. Simultaneously, when the instrument holder 280 reaches the motion boundary, the surgical robot 110 system allows a visual (e.g., indicator light) or auditory (e.g., voice) warning to alert the operator. In some embodiments, process 840 may include the operator hand-guiding dragging the instrument holder 280 movement process, or the instrument holder 280 movement process based on master-slave operation control.

Furthermore, referring to Fig. 15, which is a flowchart of an embodiment of a manual setting control method for the function of the mechanical arm 200 of the surgical robot 110 to prevent squeezing person. As shown in Fig. 15, after the instrument holder 280 completes the docking process 910 with the cannula, and before executing the step of installing the surgical instrument onto the instrument holder 280, the operator is dragging the instrument holder 280 by hands to perform rotational movement around the remote center of motion in process 920. During process 930, after the instrument holder 280 moves to the target posture. In some embodiments, the target posture may include a posture where the instrument holder 280 is close to the patient but not in touch. The operator may selectively execute process 901 to decide whether to enable the function to prevent squeezing person. If the function to prevent squeezing person is not enabled, the instrument holder 280 may continue motion control around the remote center of motion. If the function to prevent squeezing person is enabled, execute process 940: the operator needs to manually set the instrument holder 280 move to the lowest limit boundary. In some embodiments, manual setting may include pressing a button, tapping a touch screen, or other remote control methods. After completing the setting of the lowest movement limit boundary for the instrument holder 280, the current posture of the instrument holder 280 is taken as the new motion boundary value, and the surgical robot 110 system automatically executes process 950 to update the operable space of the instrument holder 280. At this time, the operator may still manually execute process 902. If the function to prevent squeezing person is turned off, the set lowest movement limit boundary for the mechanical arm 200 is canceled, and it restores to the initial boundary condition after system startup. The initial boundary is the movement range limit of each joint of the instrument holder 280 before the function to prevent squeezing person is triggered. If the function to prevent squeezing person remains on, then during movement execution process 960, the instrument holder 280 will not be allowed to continue moving in the direction beyond the set boundary but may be allowed to move in a reverse direction and in other lateral directions.

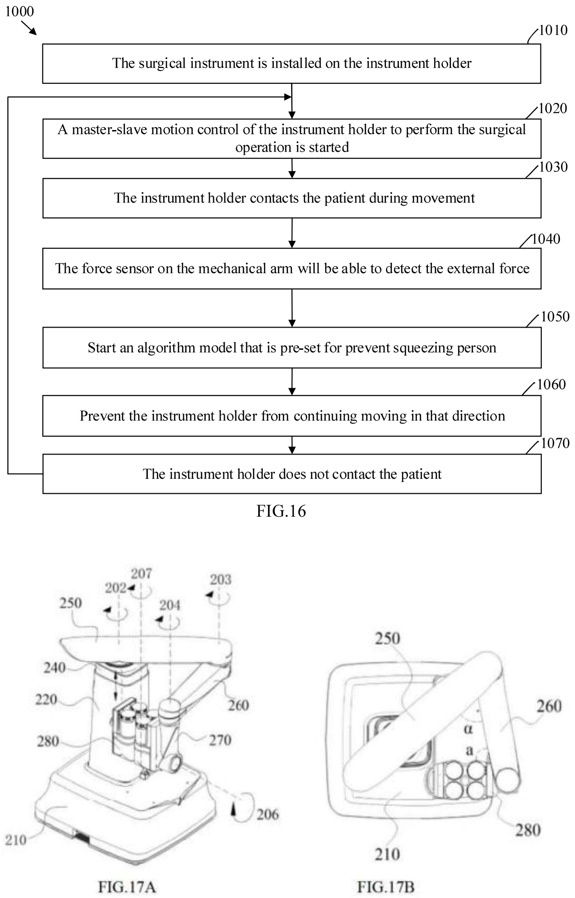

Furthermore, referring to Fig. 16, which is a flowchart of an embodiment of an automatic detection control method for the function of the mechanical arm 200 of the surgical robot 110 to prevent squeezing person. As shown in Fig. 16, after the installation process 1010 of the surgical instrument on the instrument holder 280 is completed, the lead surgeon may operate the master console manipulator to execute process 1020: master-slave motion control of the instrument holder 280 to perform the surgical operation. In process 1020, various mechanical moving parts of the instrument holder 280 execute corresponding motion commands based on the kinematic model 600. If the instrument holder 280 contacts the patient 130 during movement, the force sensor already installed on the mechanical arm 200 will be able to detect the external force in real-time. After the force sensor detects the external force, it triggers the execution of process 1050 to start an algorithm model that is pre-set for prevent squeezing person. After receiving the instruction signal, the surgical robot 110 system then executes process 1060 to prevent the instrument holder 280 from continuing moving in that direction. As the instrument holder 280 moves in the reverse direction and in other lateral directions, the instrument holder 280 does not contact the patient 130, and master-slave motion control may continue.

In practical applications, the inventors found that the above surgical robot 110 still has the following problems to be solved:
On one hand, before performing the surgery with the robot assisted, the medical staff need to do preoperative preparations. During this time, they need to push the surgical robot 110 to a suitable position next to the patient's surgical bed and operate to control the mechanical arm 200 of the surgical robot 110 to complete the preoperative initial positioning action. On the other hand, after the surgery, the surgical robot 110 also needs to be operated to control the mechanical arm 200 to execute the action of returning to the initial zero position, retracting the mechanical arm 200 for storage of the surgical robot 110. Since these are done manually, the positioning is inaccurate and inefficient.

During the movement of the mechanical arm 200 in initial positioning and postoperative retraction, the operator may cause the mechanical arm 200 to collide with an external object due to blind spots or mis-operation. This collision is an abnormal collision phenomenon, causing certain damage and destruction to the mechanical arm 200 of the surgical robot 110.

Based on this, in one aspect, referring to Figs. 1, 2, and 17A-20B, the present disclosure provides a surgical robot 110,
including: a mechanical arm 200 and a control device 290. The mechanical arm 200 includes a column 240, an upper arm 250, a forearm 260, and an instrument holder 280 connected in sequence. The instrument holder 280 is used to install the surgical instrument.

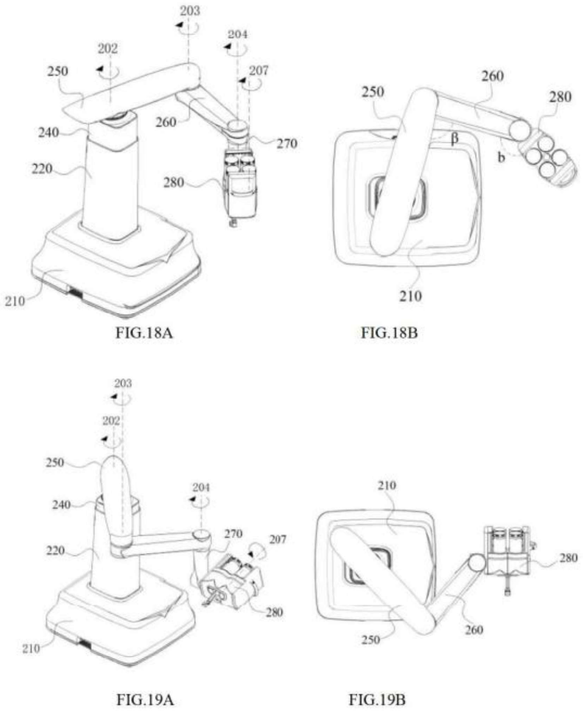

The mechanical arm 200 is further provided with a positioning input device (not shown, may be arranged on the main support column 220) for sending an input instruction to the control device 290. In response to a retraction instruction from the positioning input device, the control device 290 controls the mechanical arm 200 to move to a retracted position (as shown in Figs. 17A, 17B). In response to a drape deployment instruction from the positioning input device, the control device 290 controls the mechanical arm 200 to move to a drape deployment position (as shown in Figs. 18A, 18B).
where, the angle between the upper arm and the forearm at the retracted position (α in Fig. 17B) is smaller than the angle between the upper arm and the forearm at the drape deployment position (β in Fig. 18B); the instrument holder at the retracted position is closer to the column than at the drape deployment position.

The positioning input device may be a mechanical button on the main support column 220, or a touch screen, etc.

Where, the retracted position is the pose to be set for the surgical robot 110 before surgery starts or after surgery ends. It occupies the smallest space at this time.

Where, the drape deployment position is the position to be set for the surgical robot 110 before surgery starts, when arranging the surgical drape (sterile cloth). This pose is more conducive to wrapping the drape around the mechanical arm 200, improving operational efficiency.

Where, at the retracted position and the drape deployment position, the central axis 207 of the instrument holder is perpendicular to the horizontal plane. The central axis 207 of the instrument holder 280 passes through a center of four drive devices used to drive the surgical instrument. After the cannula is docked to the instrument holder 280, the axis of the cannula substantially coincides with the central axis 207.

Where, in response to a docking instruction from the positioning input device, the control device 290 controls the mechanical arm 200 to move to a docking position (as shown in Figs. 19A-20B). The instrument holder 280 at the retracted position is closer to the column 240 than at the docking position. At the docking position, the central axis 207 of the instrument holder 280 forms a non-right angle with the horizontal plane. The non-right angle facilitates the installation of surgical instruments and docking with the cannula.

Where, the instrument holder 280 is configured to rotate around a first axis (a pivot axis 206 in Fig. 17A). At any of the retracted position, the drape deployment position, and the docking position, the first axis (the pivot axis 206 in Fig. 17A) is perpendicular to the central axis 207 of the instrument holder.

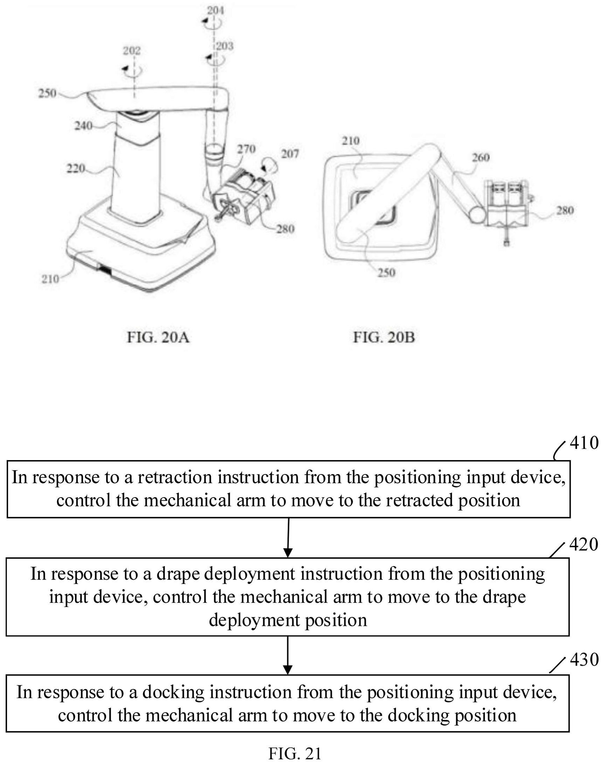

Where, the docking position includes a left docking position (as shown in Figs. 19A-19B) and a right docking position (as shown in Figs. 20A-20B). At the left docking position, the upper arm and the forearm are represented as a "<" shape. At the right docking position, the upper arm and forearm are represented as a ">" shape. The left and right docking positions are used for different operating room environments, for example, chosen based on the location of obstacles in the operating room.

Where, the mechanical arm 200 is further provided with a sensor. The sensor is configured to detect collisions experienced when the mechanical arm 200 moves from one position among the retracted position, the drape deployment position, and the docking position to another. In response to a signal from the sensor, control the mechanical arm 200 to perform anti-collision processing.

Where, the mechanical arm is further provided with a motion boundary input device. At the docking position,
in response to a motion boundary setting instruction from the motion boundary input device, the control device 290 sets current pose of the instrument holder 280 as the motion boundary.
in response to moving to the motion boundary, control the mechanical arm 200 to perform anti-collision processing.

Where, the anti-collision processing includes: the mechanical arm 200 ceasing movement, or the mechanical arm 200 moving in a predetermined manner along a direction opposite to the original movement direction. The predetermined manner includes one or more of the following motion forms: moving at a fixed speed, moving a fixed distance, or moving with a variable speed like a spring.

Where, the motion boundary input device is arranged on the instrument holder.

In this embodiment, by using the input instruction from the positioning input device to control the positioning of the mechanical arm 200, replacing dragging the mechanical arm 200 manually, and an automatic positioning functionality is achieved, avoiding inaccuracies caused by manual operation, improving efficiency, and enhancing safety and reliability of the operation of the surgical robot.

Furthermore, by setting motion boundaries for anti-collision setup, collisions of the mechanical arm 200 caused by operational errors are prevented.

Furthermore, by setting up sensors to detect collisions experienced by the mechanical arm 200 and controlling the mechanical arm 200 to perform corresponding anti-collision processing based on signals from the sensors, it may automatically respond promptly upon collision, for example, by stopping movement and waiting for the collision situation to resolve before continuing positioning.

By controlling the instrument holder 280 moves along the central axis of the cannula 150 and with the help of detecting collision based on the sensor, the probability of mechanical arm 200 collision may be reduced in specific scenarios, such as during cannula docking.

By controlling the instrument holder moves within the motion boundary range and with the help of detecting collision based on the sensor, the probability of mechanical arm 200 collision may be reduced in specific scenarios, such as during cannula docking or when adjusting the positioning of the mechanical arm 200 during surgery.

Specifically, referring to Figs. 17A-20B, which are schematic diagrams of the mechanical arm 200 structure at different positioning positions.

Among them, Fig. 17A is a front view of the retracted position of the mechanical arm 200, Fig. 17B is a top view of the retracted position. As shown, the central axes 202-204 of the joints are parallel to the central axis 207 of the instrument holder 280 (passing through the cannula central axis). The pivot axis 206 is perpendicular to the central axes 202-204. The vertical projection of the instrument holder 280 at least partially overlaps with the chassis 210. The entire mechanical arm 200 is represented as a "coiled snake" shape to reduce the space occupied by the mechanical arm 200 and the instrument holder 280.

Fig. 18A is a front view of the drape deployment position of the mechanical arm 200, Fig. 18B is a top view of the drape deployment position. As shown, a deployment angle of the mechanical arm 200 is larger than that at the retracted position: an angle between the upper arm assembly 250 and the forearm assembly 260 (β in Fig. 18B) is larger than an angle at the retracted position (α in Fig. 17B). An angle between the forearm assembly 260 and the instrument holder 280 (b in Fig. 18B) is larger than an angle at the retracted position (a in Fig. 17B).

Fig. 19A is a front view of the right docking position of the mechanical arm 200, Fig. 19B is a top view of the right docking position. As shown, the joint axes 202-204 are parallel, and the joint axes 202-204 form a non-zero angle with the central axis 207 of the instrument holder 280. The pivot axis 206 is perpendicular to the central axis 207 of the instrument holder 280. The upper arm assembly 250 and the forearm assembly 260 are represented as a "<" shape.

Fig. 20A is a front view of the left docking position of the mechanical arm 200, Fig. 20B is a top view of the left docking position. As shown, the joint axes 202-204 are parallel, and the joint axes 202-204 form a non-zero angle with the central axis 207 of the instrument holder 280. The pivot axis 206 is perpendicular to the central axis 207 of the instrument holder 280. The upper arm assembly 250 and the forearm assembly 260 are represented as a "<" shape.

On the other hand, the present disclosure provides a positioning control method for the mechanical arm of the surgical robot according to any one of the above, including steps:
410: In response to a retraction instruction from the positioning input device, control the mechanical arm to move to the retracted position.
420: In response to a drape deployment instruction from the positioning input device, control the mechanical arm to move to the drape deployment position.
where, an angle between the upper arm and the forearm at the retracted position is smaller than an angle between the upper arm and the forearm at the drape deployment position; the instrument holder at the retracted position is closer to the column than at the drape deployment position.

Furthermore, at the retracted position and the drape deployment position, control the central axis of the instrument holder to be perpendicular to the horizontal plane.

Furthermore, it also includes the step:
420: In response to a docking instruction from the positioning input device, control the mechanical arm to move to the docking position. The instrument holder at the retracted position is closer to the column than at the docking position. At the docking position, the central axis of the instrument holder forms a non-right angle with the horizontal plane.

Furthermore, the instrument holder is configured to rotate around a first axis. At any of the retracted position, the drape deployment position, and the docking position, the first axis is perpendicular to the central axis of the instrument holder.

Furthermore, the docking position includes a left docking position and a right docking position. At the left docking position, adjust the mechanical arm so that the upper arm and forearm are represented as a "<" shape. At the right positioning position, adjust the mechanical arm so that the upper arm and forearm are represented as a ">" shape.

Furthermore, in response to a signal from the sensor and/or moving to the motion boundary, control the mechanical arm to perform anti-collision processing.

Furthermore, the anti-collision processing includes: the mechanical arm ceasing movement, or the mechanical arm moving in a predetermined manner along the direction opposite to the original movement direction. The predetermined manner includes one or more of the following motion forms: moving at a fixed speed, moving a fixed distance, or moving with a variable speed like a spring.

Furthermore, when a collision abnormal signal is generated, record a collision detection log.

Furthermore, when a collision abnormal signal is generated, issue an alarm signal.

Furthermore, monitor current of each moving part of the mechanical arm. When the current becomes abnormal, generate a collision abnormal signal.

Furthermore, monitor the external force on the sensor. When the external force is detected, generate a collision abnormal signal.

Using the solution of the present disclosure, by adding collision detection and post-processing functions to the surgical robot 110, damage or destruction caused by the abnormal collision is avoided, improving the safety and reliability of the surgical robot 110 operation.

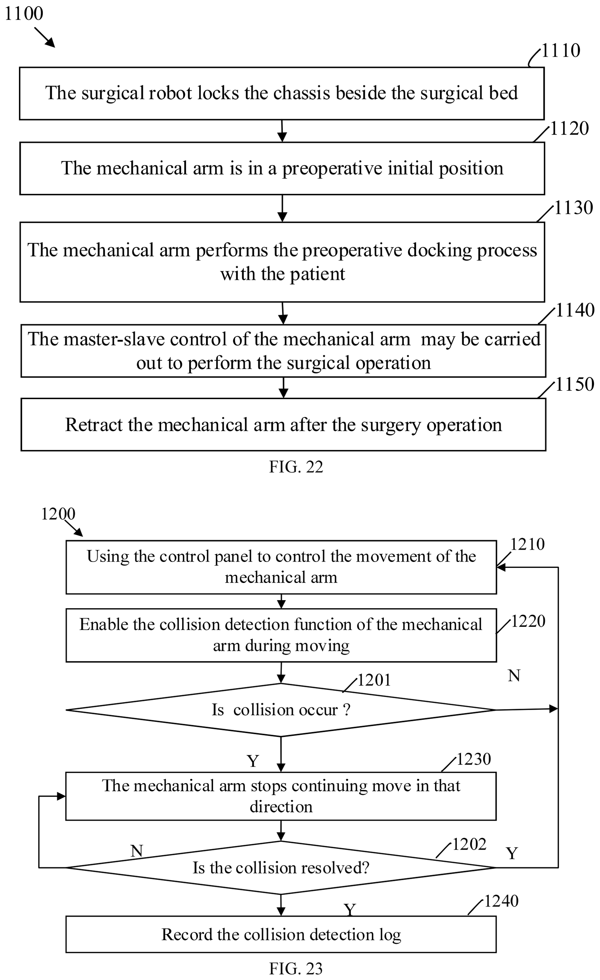

Specifically, referring to Fig. 22, Fig. 22 is a flowchart of an embodiment of a motion control process 1100 of the mechanical arm 200 of the surgical robot 110. Among them, the preoperative initial positioning process 1120 of the mechanical arm 200 is usually completed by the operator through the control panel. The operator continuously adjusts the movement of the mechanical arm 200 based on surgical procedure requirements and an operable space of the mechanical arm 200 to achieve an intended positioning target. In some embodiments, displacement sensors are installed at joints of the mechanical arm 200 to obtain motion information in real-time. The joints are located at prismatic and revolute positions of moving parts of the mechanical arm 200. In some embodiments, each moving part has its own motion coordinate system established, and coordinate transformation formulas (such as homogeneous transformation formulas) are applied to establish motion transformation relationships between adjacent coordinate systems and between the end of the mechanical arm 200 and the base reference coordinate system. In some embodiments, applying forward kinematic transformation using the established combination of coordinate systems may obtain pose information of the end of the mechanical arm 200 relative to the base reference coordinate system. Applying inverse kinematic transformation using the established combination of coordinate systems may obtain the motion information of each moving part required for the end of the mechanical arm 200 to reach the expected target. After completing the preoperative positioning of the mechanical arm 200, the medical staff may drag the mechanical arm 200 by hand to perform the preoperative docking process 1130 with the patient 130. After the surgical instruments are installed, master-slave control of the mechanical arm 200 may be carried out to perform the surgical operation process 1140. During this movement, there is usually some relative force (such as friction) between the end of the mechanical arm 200 and the patient 130 at the patient incision location 170. After surgery, to facilitate compact storage of the surgical robot 110, the medical staff usually use the control panel to control the mechanical arm 200 to execute the motion retraction process 1150, retracting the mechanical arm 200.

Fig. 23 is a simplified flowchart of a collision detection method 1200 for the mechanical arm 200 of the surgical robot 110. The process 1210 of the operator using the control panel to control the movement of the mechanical arm 200 includes the preoperative positioning process 1120 and the postoperative retraction process 1150 of the mechanical arm 200. During this movement, the program automatically enables the collision detection function 1220 of the mechanical arm 200. In some embodiments, multi-dimensional force/torque sensors are allowed to be designed and installed at the motion joint positions of the moving parts of the mechanical arm 200 for motion control based on mechanical principles. Furthermore, the installed multi-dimensional force/torque sensors can monitor whether collisions occur during the movement of the mechanical arm 200. In some embodiments, methods for detecting whether the mechanical arm 200 collides with an external object include but are not limited to: monitoring whether current of each moving part of the mechanical arm 200 has a sudden abnormal change, or monitoring whether the measurement data of the installed multi-dimensional force/torque sensors has a sudden abnormal change. During the movement of the mechanical arm 200, if the output signal of process 1201 is "No", the mechanical arm 200 is allowed to continue moving in that direction. If the output signal of process 1201 is "Yes", execute process 1230: the mechanical arm 200 stops continuing move in that direction. In some embodiments, when a collision is detected, the robot system allows alerting the operator to promptly terminate the operation causing the collision through voice broadcast, icon display, indicator light flashing or color change, interrupting motion command transmission, etc., so that the operator may discover and perform operations to resolve the collision in time, avoiding further damage to the surgical robot 110 and the collided object. In some embodiments, methods to stop the mechanical arm 200 from continuing move in that direction include but are not limited to immediately terminating motion commands or automatically executing pre-programmed motion commands using the position where the mechanical arm 200 collided as a reference. In some embodiments, the specific execution actions of automatically executing pre-programmed motion commands using the collision position as the reference may include moving at a fixed speed, moving a fixed distance, or moving with a variable speed like a spring. Furthermore, concurrently execute process 1202: judge in real-time whether the collision of the mechanical arm 200 is resolved. If the output signal of process 1202 is "No", continue executing process 1230. If the output signal of process 1202 is "Yes", then process 1210 may be executed to continue controlling the movement of the mechanical arm 200. Simultaneously, the robot system automatically executes the process 1240 of recording the collision detection log. In some embodiments, the collision detection log may be stored in the robot system in a readable format such as text or charts for abnormal recording and later abnormal collision diagnosis.

In yet another aspect, the present disclosure provides a computer-assisted surgical system, including the surgical robot 110 according to any one of the above. The computer-assisted surgical system further includes a remote console.

In still another aspect, the present disclosure provides a readable storage medium. The readable storage medium stores a computer program which, when executed by a processor, implements the steps of the various methods for the surgical robot 110 described above.

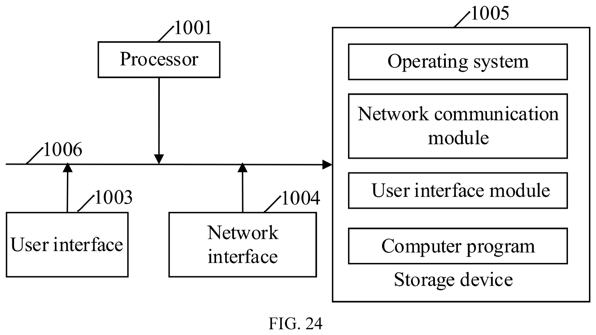

Specifically, referring to Fig. 24. In practical applications, Fig. 24 is a schematic diagram of the structure of a hardware operating environment involved in the various methods of the surgical robot 110 of the present disclosure.

As shown in Fig. 24, the hardware operating environment may include: a processor 1001, such as a CPU; a communication bus 1002; a user interface 1003; a network interface 1004; and a storage device 1005. The communication bus 1002 is used to implement connection and communication between these components. The user interface 1003 may include a display, input units such as a keyboard, gimbal, etc. Optionally, the user interface 1003 may also include standard wired and wireless interfaces. The network interface 1004 may optionally include standard wired and wireless interfaces (e.g., WI-FI interface). The storage device 1005 may be high-speed RAM memory, or stable memory (non-volatile memory), such as disk storage. Optionally, the storage device 1005 may also be a storage device independent of the afore-mentioned processor 1001.

Those skilled in art may understand that the hardware structure for running the various methods of the surgical robot 110 shown in Fig. 24 does not constitute a limitation on the device running these methods. It may include more or fewer components than illustrated, or combine some components, or have a different arrangement of components.

As shown in Fig. 24, the storage device 1005, as a readable storage medium, may include an operating system, a network communication module, a user interface module, and a program for automatic direction setting of the surgical robot 110. The operating system manages and controls the hardware, supporting the operation of the network communication module, user interface module, programs for the various control methods of the surgical robot 110, and other programs or software. The network communication module is used to manage and control the network interface 1004. The user interface module is used to manage and control the user interface 1003.

In the hardware structure shown in Fig. 24, the network interface 1004 is mainly used to connect to a background server and perform data communication with it. The user interface 1003 is mainly used to connect to a client (user end) and perform data communication with it. The processor 1001 may invoke the programs for the various control methods of the surgical robot 110 stored in the storage device 1005.

In summary, the present disclosure has the following beneficial effects: Using the solution of the present disclosure,

By controlling the surgical robot 110 to enable the mechanical arm 200 perform docking movements, including free movement, rotation around the remote center of motion, or translational movement at different stages, efficient and accurate completion of the docking operation with the cannula is achieved, improving the safety of the surgical robot's operation and reducing operational risks.

Furthermore, the present disclosure uses free movement to complete the movement of the instrument holder 280 over large distances and rotation ranges, e.g., dragging it above the patient's 130 surgical location. Rotation around the remote center of motion 160 completes the alignment of the docking device 140 on the instrument holder 280 with the cannula 150. Finally, translation completes the connection between the docking device 140 and the cannula 150. Moreover, the types and execution order of the above docking movements may be changed according to the actual clinical experience of the doctor. Through the combination of different types of docking movements, the precision and efficiency of the docking operation are further improved.

Furthermore, corresponding to the above movements, the first input device 2802 is provided on the handle 2801. When grabbing the handle 2801 to drag the instrument holder 280, one may conveniently press the first input device 2802 on the handle 2801 to select different types of docking movements, allowing flexible switching and control with one hand.

Furthermore, by setting a force sensor on the handle 2801, the force applied by the hand is perceived more accurately. Compared to force sensors placed in other locations, this allows for more precise docking operations and reduces danger.

Furthermore, by setting multiple force sensors on the instrument holder 280 and setting up different coordinate systems corresponding to multiple origins on the instrument holder 280, and cleverly arranging them at corresponding positions, different force sensors and coordinate systems may be used to control the docking movement of the instrument holder 280 for different scenarios, further refining the docking operation.

Furthermore, by providing a direct docking second input device 2805 on the docking device 140, the medical staff may directly grab the docking device 140 to drag and complete the docking movement. Compared to grabbing the handle 2801 and dragging, this makes the cannula 150 docking process more direct. By reducing the speed of translational movement, the translation docking process may be controlled more finely.

Furthermore, based on whether the docking device 140 is connected to the cannula 150 and whether the surgical instruments are installed, fine-grained scenario management is performed. Different scenarios allow the use of different types of docking movements. For example, as the risk increases, the range of docking movements is limited, and the speed is reduced, further reducing surgical risk.

Furthermore, providing a docking movement method that allows the instrument holder 280 to perform automatic translational movement along the central axis 180 direction of the cannula 150 preoperatively and intraoperatively, combined with adjustments to the remote center of motion 160, etc., meets the needs of specific scenarios where patients 130 have individual differences or the lesion H is in different positions.

Furthermore, for the scenario where the lesion H is too close to the human skin, requiring the cannula 150 to be pulled out of the body, a corresponding incision protector 190 is designed. This ensures that after adjusting the remote fixed point, it may also prevent the human incision 170 from being scratched by surgical instruments and prevent intra-abdominal gas leakage.

Furthermore, the dragging control function by hand for the mechanical arm 200 of the surgical robot 110 is realized, including 5-DOF dragging control, 3-DOF translational dragging control, 2-DOF fixed-point rotational dragging control, forward/backward directional dragging control along the cannula 150 axis, and jog button control along the cannula 150 axis. This facilitates flexible positioning of the mechanical arm 200 during preoperative preparations and quick and smooth docking operations with the cannula 150, expands more application scenarios, requires no manual setup, and improves work efficiency.

Furthermore, by adding the function of intraoperatively preventing the mechanical arm 200 from squeezing the patient to the surgical robot 110, secondary injury to the patient caused by abnormal collisions is avoided, improving the safety and reliability of the surgical robot 110 operation.

Furthermore, by adding a positioning input device to the surgical robot 110 for sending input instructions to the control device, automatic positioning functionality is achieved, avoiding inaccuracies caused by manual operation, improving efficiency, and enhancing the safety and reliability of the surgical robot's operation.

Furthermore, by adding collision detection and post-processing functions during the positioning of the surgical robot 110, damage or destruction caused by abnormal collisions is avoided, improving the safety and reliability of the surgical robot 110 operation.

## Claims

1. A surgical robot, comprising:
a mechanical arm and a control device, wherein an end of the mechanical arm is provided with an instrument holder for installing a plurality of surgical instruments, the control device is configured to control the mechanical arm to move and enable the plurality of surgical instruments rotate around a same remote center of motion, and:
in response to a boundary setting instruction, set a current posture of the instrument holder as a motion boundary when the instrument holder moves around the remote center of motion;
in response that the instrument holder rotates around the remote center of motion to the motion boundary, control the instrument holder to move in a preset motion mode.

2. The surgical robot according to claim 1, wherein the control device is further configured to:
in response to a reconfiguration instruction, reconfigure a distance from the remote center of motion to the instrument holder along a direction of a central axis of the instrument holder, the instrument holder rotates around a reconfigured remote center of motion.

3. The surgical robot according to claim 2, wherein the control device is further configured to: in response that the distance from the remote center of motion to the instrument holder is configured to be decreased, decrease the motion boundary correspondingly within a safe range; in response that the distance from the remote center of motion to the instrument holder is configured to be increased, increase the motion boundary correspondingly within a safe range.

4. The surgical robot according to claim 2, wherein the control device is further configured to: in response that the distance from the remote center of motion to the instrument holder is reconfigured, set the motion boundary to be invalid.

5. The surgical robot according to claim 2, wherein reconfiguring distance from the remote center of motion to the instrument holder comprises modifying a transformation relationship from a coordinate system of the remote center of motion to a base coordinate system.

6. The surgical robot according to claim 5, wherein the modifying the transformation relationship from the coordinate system of the remote center of motion to the base coordinate system comprises: translating the coordinate system of the remote center of motion along the central axis of the instrument holder.

7. The surgical robot according to claim 1, wherein the control device is further configured to: in response to a translation of the instrument holder or rotation not around the remote center of motion, a position of the remote center of motion relative to the base coordinate system is unchanged by adjusting the mechanical arm.

8. The surgical robot according to claim 1, wherein the surgical robot further comprises an identification device that identifies an incision position of a human body, the distance from the remote center of motion to the instrument holder is relatively fixed and configurable, the control device is configured to: control the instrument holder to move until an origin of the coordinate system of the remote center of motion is coincide with an origin of an incision coordinate system, the incision coordinate system being a coordinate system established by the control device based on the incision position of the human body identified by the identification device.

9. The surgical robot according to claim 1, wherein the mechanical arm is provided with a sensor, the sensor is configured to detect squeezing when the mechanical arm contacts the human body, the control device is further configured to, in response to a signal from the sensor, control the mechanical arm to perform an anti-squeezing processing, the anti-squeezing processing comprising: in response that the mechanical arm squeezing the human body, control the instrument holder to move in a preset motion mode.

10. The surgical robot according to claim 9, wherein,
the sensor comprises a first sensor, the first sensor is arranged at a joint between the instrument holder and the mechanical arm, the sensor is a non-contact sensor.

11. The surgical robot according to claim 9, wherein the sensor comprises a second sensor, the second sensor is arranged on bottom surface of the instrument holder, the sensor is a contact sensor.

12. The surgical robot according to claim 9, wherein
in response that an external force is greater than a preset first threshold, the control device generates a squeeze warning instruction;
in response that the external force is less than a preset second threshold, the control device generates a warning release instruction.

13. The surgical robot according to any one of claims 1-12, wherein moving in the preset motion mode comprises: moving along other directions different from the current motion direction, or ceasing movement.

14. The surgical robot according to any one of claims 2-8, wherein the surgical robot further comprising a cannula coupled to the instrument holder, the reconfigured remote center of motion is located on an axis of the cannula and outside the cannula.

15. The surgical robot according to claim 14, wherein the surgical robot further comprising a flexible incision protector, a proximal end of the incision protector is used to connect to the cannula, a distal end of the incision protector is used to connect to the incision of the human body, the remote center of motion is located within the incision protector.

16. The surgical robot according to any one of claims 1-12, wherein the surgical robot further comprising: a base and the instrument holder, wherein a proximal end of the mechanical arm is connected to the base, a distal end of the mechanical arm is connected to the instrument holder, the instrument holder is used for mounting the surgical instruments and docking with the cannula, the instrument holder is configured to be rotatable around the remote center of motion, and the control device is configured to:
in response to a first docking instruction, control the mechanical arm to move to enable the instrument holder to move freely under the external force, thereby rotating or translating the remote center of motion;
in response to a second docking instruction, control the mechanical arm to move to maintain the remote center of motion stationary relative to the base, and allow the instrument holder to rotate around the remote center of motion at a first speed under the external force, while prohibiting translational movement of the instrument holder along three axes of a Cartesian coordinate system, a magnitude of the first speed being associated with a magnitude of the external force applied to the instrument holder;
in response to a third docking instruction, control the mechanical arm to move to allow the instrument holder to translate along the three axes of the Cartesian coordinate system at a second speed under the external force, while prohibiting rotation of the instrument holder around the remote center of motion, a magnitude of the second speed being associated with the magnitude of the external force applied to the instrument holder.

17. The surgical robot according to any one of claims 1-12, wherein the mechanical arm comprises a column, an upper arm, a forearm, and the instrument holder connected in sequence, the instrument holder is used to install the surgical instruments;
the mechanical arm is further provided with a positioning input device for sending an input instruction to the control device, in response to a retraction instruction from the positioning input device, the control device controls the mechanical arm to move to a retracted position, in response to a drape deployment instruction from the positioning input device, the control device controls the mechanical arm to move to a drape deployment position;
wherein, an angle between the upper arm and the forearm at the retracted position is smaller than an angle between the upper arm and the forearm at the drape deployment position; the instrument holder at the retracted position is closer to the column than the instrument holder at the drape deployment position.

18. A method for controlling anti-squeeze of the mechanical arm of the surgical robot according to any one of claims 1-17, comprising:
in response to a boundary setting instruction, setting the current posture of the instrument holder as a motion boundary for the movement of the instrument holder around the remote center of motion;
in response to the instrument holder rotating around the remote center of motion to the motion boundary, controlling the instrument holder to move in the preset motion mode.

19. A computer-assisted surgical system, comprising a surgical robot according to any one of claims 1-17 and a console for remotely controlling the surgical robot.

20. A readable storage medium, wherein the readable storage medium has stored there on a computer program, which when executed by a processor, implements steps of the method for controlling anti-squeeze of the mechanical arm of the surgical robot according to claim 18.
